# EUROPEAN PATENT APPLICATION

(11) **EP 4 241 717 A1**
(43) Date of publication of application: **13.09.2023**
(21) Application number: 21876865.3
(22) Date of filing: 10.05.2021
(51) Int. Cl.: A61B 34/37, G06K 9/00, G06N 3/04, G06T 7/246

(54) **SURGICAL ROBOT AND MOTION ERROR DETECTION METHOD AND DETECTION DEVICE THEREFOR**

(30) Priority: 08.10.2020 CN 202011068075
(71) Applicant: Shenzhen Edge Medical Co., Ltd., Shenzhen, Guangdong 518000 (CN)
(72) Inventor: GAO, Yuanqian, Shenzhen, Guangdong 518000 (CN); WANG, Jianchen, Shenzhen, Guangdong 518000 (CN); WANG, Pai, Shenzhen, Guangdong 518000 (CN)
(74) Representative: Zaboliene, Reda
(86) International application number: PCT/CN2021/092542
(87) International publication number: WO 2022/073342

(57) **Abstract**

A surgical robot and a motion error detection method and detection device therefor. The surgical robot includes a camera arm, a surgical arm, an input part (21), and a control device coupled to the camera arm, the surgical arm, and the input part (21). The control device is configured to: acquire an operation image of surgical area acquired by the camera arm (S11); if the operation image contains a sub-image of the surgical arm, recognize a feature part of the surgical arm from the sub-image as a first feature part (S12); acquire a control command inputted by the input part (21), obtain a kinematic model of the surgical arm according to the control instruction (S13); obtain a second feature part matching the first feature part from the kinematic model (S14); obtain actual motion information of the first feature part and target motion information of the second feature part (S15); compare the actual motion information with the target motion information to determine whether the surgical robot has a motion error (S16). The surgical robot can at least detect whether the surgical robot has a motion error.

## Description

### CROSS-REFERENCES TO RELATED APPLICATIONS

The present disclosure claims a priority of the Chinese patent application No.CN202011068075.5, filed on October 8, 2020 in China National Intellectual Property Administration, entitled "SURGICAL ROBOT AND MOTION ERROR DETECTION METHOD AND DETECTION DEVICE THEREFOR", the entire contents of which are incorporated in the present disclosure.

### FIELD

The present application relates to the field of medical devices, in particular to a surgical robot, and a motion error detection method and detection device thereof.

### BACKGROUND

Minimally invasive surgery refers to a surgical method of performing a procedure in a human body cavity using modern medical instruments such as laparoscopes, thoracoscopes, and so on. Compared with traditional surgery modes, minimally invasive surgery has advantages of being in little trauma, little pain, fast recovery, and the like.

With advances in science and technology, minimally invasive surgical technologies are increasingly mature and widely used. Surgical robots usually include a master console and a slave operating device, the master console includes a display and a input unit, the slave operation device includes a plurality of robot arms, one of the robot arm is used to obtain the image of the surgical field, and the image is then displayed by the display. The other robot arms is used to perform surgical operation.

In the master-slave control mode, the input device sends control commands, which are received and executed by the controlled robot arm. However, due to the poor assembly accuracy and/or transmission accuracy of the input device and/or the robot arm itself, it is easy to cause that the control commands input by the doctor to cannot be accurately executed by the robot arm, likely resulting surgical risks. Detecting whether there is a motion error in the robot arm has become an urgent problem to be solved.

### SUMMARY

Based on this, it is necessary to provide a surgical robot and motion error detection method and detection device therefor.

In one aspect, a surgical robot is provided. The surgical robot comprises a camera arm, at least one operating arm having more than one feature site, an input device configured for receiving at least one control command for controlling the camera arm and/or the at least one operating arm to move, and a control device operatively coupled to the camera arm and the at least one operating arm, the control device is configured for: obtaining at least one operational image of a surgical field captured by the camera arm; identifying at least one of the feature sites of the at least one operating arm as at least one first feature site; obtaining the at least one control command from the input device, and obtaining a kinematics model of the at least one operating arm according to the at least one control command; obtaining at least one second feature site matching the at least one first feature site in the kinematics model; obtaining actual motion information of the at least one first feature site, and obtaining target motion information of the at least one second feature site; and comparing the actual motion information with the target motion information to determine whether there is a motion error in the surgical robot.

In one embodiment, in the identifying the feature site of the at least one operating arm from the at least one operational image, the control device is configured for: detecting whether at least one sub-image is comprised in the at least one operational image, and each of the at least one sub-image is at least part of the at least one operating arm; and extracting the at least one sub-image from the at least one operational image in response to the at least one sub-image being detected; identifying the feature site of the at least one operating arm based on the at least one sub-image.

In one embodiment, in the detecting whether at least one sub-image is comprised in the at least one operational image, the control device is configured for: inputting the at least one operational image into a neural network, and determining whether the at least one sub-image of the at least one operating arm is comprised in the at least one operational image through the neural network.

In one embodiment, in the identifying the feature site of the at least one operating arm based on the at least one sub-image, the control device is configured for: inputting the at least one sub-image into a neural network, and detecting the feature site of the at least one operating arm based on the at least one sub-image through the neural network.

In one embodiment, in the identifying the feature site of the at least one operating arm from the at least one operational image, the control device is configured for: inputting at least one operational image into a neural network, and detecting the feature sites of at least one operating arm based on at least one operational image through the neural network.

In one embodiment, in the obtaining the actual motion information of the at least one first feature site, the control device is configured for: predicting the actual motion information of the at least one first feature site through the neural network.

In one embodiment, the neural network is a convolutional neural network.

In one embodiment, before the predicting the actual motion information of the at least one first feature site through the neural network, the control device is configured for: training the convolutional neural network according to a difference between a prediction result and a real result, wherein the prediction result corresponds to the feature image extracted from a preset training-set through the convolutional neural network, and the real result corresponds to the preset training-set.

In one embodiment, the actual motion information comprises actual position information and/or actual pose information.

In one embodiment, the actual motion information comprises actual velocity information and/or actual angular velocity information.

In one embodiment, an image end instrument with binocular vision is coupled to a distal end of the camera arm; in the obtaining the actual motion information of the at least one first feature site, the control device is configured for: obtaining the actual motion information of the at least one first feature site under a viewpoint of image end instrument through a parallax principle, wherein the actual motion information is actual position information and/or orientation information.

In one embodiment, in the obtaining the target motion information of the at least one second feature site, the control device is configured for: calculating the target motion information of the at least one second feature site according to the kinematics model, wherein the target motion information is target position information and/or target orientation information.

In one embodiment, in the obtaining the kinematics model of the at least one operating arm according to the at least one control command, the control device is configured for: obtaining target pose information of a distal end of the at least one operating arm by analyzing the at least one control command; obtaining at least one joint variable of each joint assembly of the at least one operating arm by analyzing the target pose information; and calculating the kinematics model of the at least one operating arm according to the at least one joint variable through a forward kinematics equation.

In one embodiment, in the comparing the actual motion information with the target motion information to determine whether there is the motion error in the surgical robot, the control device is configured for: calculating a deviation between the actual motion information and the target motion information; judging whether the deviation exceeds a pre-set range; and determining that there is a motion error in the surgical robot in response to the deviation exceeding the pre-set range; determining that there is no motion error in the surgical robot in response to the deviation not exceeding the pre-set range.

In one embodiment, after the determining that there is a motion error in the surgical robot, the control device is configured for: generating at least one motion error warning message.

In one embodiment, the surgical robot comprises a display coupled to the control device, after the generating the at least one motion error warning message, the control device is configured for: displaying the at least one motion error warning message on the display.

In one embodiment, in the displaying the at least one motion error warning message on the display, the control device is configured for: generating at least one warning identification corresponding to the at least one motion error warning message in the at least one sub-image and displaying the at least one warning identification on the display.

In one embodiment, the at least one warning identification comprises at least one contour line, and in the generating the at least one warning identification corresponding to the at least one motion error warning message in the at least one sub-image, the control device is configured for: generating the at least one contour line at a contour of the at least one sub-image.

In one embodiment, the surgical robot comprises an audio device coupled to the control device, after the generating the at least one motion error warning message, the control device is configured for: generating auditory feedback corresponding to the at least one motion error warning message by the audio device.

In one embodiment, the surgical robot comprises a haptic device coupled to the control device, after the generating the at least one motion error warning message, the control device is configured for: generating haptic feedback corresponding to the at least one motion error warning message by the haptic device.

In one embodiment, the haptic device is a vibration device, and the haptic feedback is reflected by a vibration; or the haptic device is a resistance device, and the haptic feedback is reflected by a resistance force.

In one embodiment, in the generating haptic feedback corresponding to the at least one motion error warning message by the haptic device, the control device is configured for: generating the haptic feedback corresponding to the at least one motion error warning message, wherein the haptic feedback is positively correlated with a degree of the deviation exceeding the pre-determined range.

In one embodiment, after the determining that there is a motion error in the surgical robot, the control device is configured for: generating at least one control command for forbidding a movement of the at least one operating arm to prevent the at least one operating arm from being controlled to move; or generating at least one control command configured to prevent an input from the input device.

In one embodiment, the input device is a mechanical handle, and each joint of the mechanical handle is equipped with a motor which drives the joint to move, after the determining that there is a motion error in the surgical robot, the control device is configured for: generating at least one control command of increasing a torsion and/or torque of each motor to form a resistance force which prevents a movement of the mechanical handle.

In one embodiment, before the obtaining the at least one second feature site(s) matching the at least one first feature site(s) in the kinematics model, the control device is configured for: obtaining a weight value of each of the at least one first feature site; weighting the first feature site according to the weight value of the first feature site to obtain a weighted value; judging whether the weighted value reaches a start-up threshold; and obtaining the at least one second feature site matching the at least one first feature site in the kinematics model in response to the weighted value reaching the start-up threshold.

In one embodiment, each feature site comprises at least one prominent feature point which facilitates the identifying of the at least one feature site.

In one embodiment, the at least one feature point comprises at least one of plane pattern, stereo geometry, and color.

In one embodiment, the at least one feature point comprises at least one of point, line, and plane.

In one embodiment, in one embodiment, the at least one feature point comprises a graphic code with a pattern, and there is a correlation between the pattern and the feature sites, so that the at least one joint variable of the associated feature sites is obtained by calculating a zoomed state and/or a rotation state of the pattern under the viewpoint of the camera arm.

In one embodiment, the at least one feature point comprises at least one of point, line, and plane.

In one embodiment, different feature sites are characterized by at least one different feature point.

In one embodiment, the feature sites comprise at least one of joint, linkage, and end instrument.

In another aspect, a surgical robot is provided. The surgical robot, includes a camera arm, at least one operating arm having more than one feature site, an input device for inputting at least one control command for controlling the camera arm and/or the at least one operating arm to move, and a control device operatively coupled to the camera arm and the at least one operating arm, the control device is configured for: obtaining at least one operational image of a surgical field captured by the camera arm; identifying feature sites of the at least one operating arm of the at least one operational image as at least one first feature site; obtaining the at least one control command from the input device, and obtaining a kinematics model of the at least one operating arm according to the at least one control command; obtaining at least one second feature site matching the at least one first feature site in the kinematics model; and displaying the at least one second feature site on the display, comparing the at least one second feature site with the at least one first feature site to determine whether there is a motion error in the surgical robot.

In one embodiment, after identifying the feature sites of the at least one operating arm from the at least one operational image, the control device is configured for: generating at least one first feature site identification identifying the at least one first feature site on the display.

In one embodiment, in the generating the at least one first feature site identification on the display, the control device is configured for: generating the at least one first feature site identification identifying the at least one first feature site in at least one sub-image.

In one embodiment, an image end instrument with binocular vision is coupled to a distal end of the camera arm, in the generating the at least one first feature site identification identifying the at least one first feature site on the display, the control device is configured for: obtaining actual position information and/or orientation information of the at least one first feature site under a viewpoint of image end instrument through a parallax principle; reconstructing a 3D image of the at least one first feature site under the viewpoint of image end instrument based on the actual position information and/or posture information of the at least one first feature site and structural feature description information corresponding to the at least one first feature site; and generating the at least one first feature site identification identifying the at least one first feature site in the 3D image, and the at least one first feature site identification is at a position corresponding to the at least one first feature site.

In one embodiment, in the generating the at least one first feature site identification on the display, the control device is configured for: obtaining position information and/or posture information of the at least one first feature site through a neural network; reconstructing 3D image of the at least one first feature site under the viewpoint of image end instrument based on the actual position information and/or posture information of the at least one first feature site and structural feature description information corresponding to the at least one first feature site; and generating at least one first feature site identification identifying the at least one first feature site in the 3D image, and the at least one first feature site identification is at a position corresponding to the at least one first feature site.

In one embodiment, in the displaying the at least one second feature site on the display, the control device is configured for: generating a model of the at least one second feature site under the viewpoint of image end instrument based on the calculated kinematics model of the at least one operating arm and structural feature description information of the at least one second feature site; and generating at least one second feature site identification identifying the at least one second feature site in the model, and the at least one second feature site identification is at a position corresponding to the at least one second feature site.

In one embodiment, in the identifying feature sites of the at least one operating arm from the at least one operational image, the detection method includes: inputting the at least one operational image into a neural network, and detecting feature sites of the at least one operating arm based on at least one operational image through the neural network.

In one embodiment, after the detecting feature sites of the at least one operating arm based on the at least one operational image through the neural network, the control device is configured for: predicting actual pose information of the feature sites through the neural network; calculating a spacial model of an effective part of the feature sites based on the actual pose information of feature sites and a predetermined range; generating a motion boundary to identify the feature sites, wherein the motion boundary is transformed from the spatial model, and the motion boundary is at a position corresponding to the feature sites in the at least one sub-image under the viewpoint of the image end instrument.

In one embodiment, the motion boundary of the feature sites comprises a plurality of substantially independent contours in response to the feature sites in the at least one operating arm being discontinuous parts; and the motion boundary of the feature sites comprises a whole contour in response to the feature sites in the at least one operating arm being continuous parts.

In one embodiment, after the generating the motion boundary to identify the feature sites, the control device is configured for: obtaining a first area of the motion boundary of the at least one first feature site under the viewpoint of image end instrument; obtaining a second area of the at least one second feature site under the viewpoint of image end instrument, and the at least one second feature site is within or beyond the motion boundary of the at least one first feature site ; calculating a ratio of the second area with respect to the first area, and displaying the ratio on the display for determining whether there is a motion error in the surgical robot.

In one embodiment, before the obtaining the at least one second feature site matching the at least one first feature site in the kinematics model, the control device is configured for: obtaining a weight value of each of the first characteristics site; weighting each of the first feature site according to the weight value of the first feature site to obtain a weighted value; judging whether the weighted value reaches a start-up threshold; and obtaining the at least one second feature site matching the at least one first feature site in the kinematics model in response to the weighted value reaching the start-up threshold.

In another aspect, a surgical robot is provided. The surgical robot includes a camera arm having a first image end instrument, at least one operating arm having more than one feature site, a second image end instrument, an input device configured for receiving at least one control command for controlling the camera arm and/or the at least one operating arm to move, and a control device operatively coupled to the camera arm, the at least one operating arm, the second image end instrument and the input device, the control device is configured for: obtaining at least one monitoring image of the camera arm and/or the at least one operating arm captured by the second image end instrument; identifying at least one of the feature sites of the camera arm and/or the at least one operating arm of the at least one monitoring image as at least one first feature site; obtaining the at least one control command from the input device, and obtaining a kinematics model of the at least one operating arm according to the at least one control command; obtaining at least one second feature site matching the at least one first feature site in the kinematics model; obtaining actual motion information of the at least one first feature site, and obtaining target motion information of the at least one second feature site; and comparing the actual motion information with the target motion information to determine whether there is a motion error in the camera arm and/or the at least one operating arm.

In another aspect, a surgical robot is provided. The surgical robot includes a camera arm having a first image end instrument, at least one operating arm having more than one feature site, a second image end instrument having with respect to the camera arm and the at least one operating arm, an input device for inputting at least one control command for controlling the camera arm and/or the at least one operating arm to move, and a control device operatively coupled to the camera arm, the at least one operating arm, the second image end instrument and the input device, the control device being configured for: obtaining at least one monitoring image of the camera arm and/or the at least one operating arm captured by the second image end instrument; identifying feature sites of the camera arm and/or the at least one operating arm of the at least one monitoring image as at least one first feature site; obtaining the at least one control command from the input device, and obtaining a kinematics model of the at least one operating arm according to the at least one control command; obtaining at least one second feature site matching the at least one first feature site in the kinematics model; and displaying the at least one second feature site on the display, comparing the at least one second feature site with the at least one first feature site to determine whether there is a motion error in the camera arm and/or the at least one operating arm.

In another aspect, a surgical robot is provided. The surgical robot includes a camera arm, at least one operating arm having more than one feature site, an input device configured for receiving at least one control command for controlling the camera arm and/or the at least one operating arm to move, comprising: obtaining at least one operational image of a surgical field captured by the camera arm; identifying at least one of the feature sites of the at least one operating arm of the at least one operational image as at least one first feature site; obtaining the at least one control command from the input device, and obtaining a kinematics model of the at least one operating arm according to the at least one control command; obtaining at least one second feature site matching the at least one first feature site in the kinematics model; obtaining actual motion information of the at least one first feature site, and obtaining target motion information of the at least one second feature site; and comparing the actual motion information with the target motion information to determine whether there is a motion error in the surgical robot.

In another aspect, a motion error detection method is provided. The motion error detection method for a surgical robot, wherein the surgical robot comprises a display, a camera arm, at least one operating arm having more than one feature site and an input device configured for receiving at least one control command for controlling the camera arm and/or the at least one operating arm to move, comprises: obtaining at least one operational image of a surgical field captured by the camera arm; identifying at least one of the feature sites of the at least one operating arm of the at least one operational image as at least one first feature site; obtaining the at least one control command from the input device, and obtaining a kinematics model of the at least one operating arm according to the at least one control command; obtaining at least one second feature site matching the at least one first feature site in the kinematics model; displaying the at least one second feature site on the display, comparing the at least one second feature site with the at least one first feature site to determine whether there is a motion error in the surgical robot.

In another aspect, a motion error detection method is provided. The motion error detection method for a surgical robot, wherein the surgical robot comprises a camera arm having a first image end instrument, at least one operating arm having more than one feature site, a second image end instrument having with respect to the camera arm and the at least one operating arm, and an input device configured for receiving at least one control command for controlling the camera arm and/or the at least one operating arm to move, comprises: obtaining at least one monitoring image of the camera arm and/or the at least one operating arm captured by the second image end instrument; identifying at least one of the feature sites of the camera arm and/or the at least one operating arm of the at least one monitoring image as at least one first feature site; obtaining the at least one control command from the input device, and obtaining a kinematics model of the at least one operating arm according to the at least one control command; obtaining at least one second feature site matching the at least one first feature site in the kinematics model; obtaining actual motion information of the at least one first feature site, and obtaining target motion information of the at least one second feature site; and comparing the actual motion information with the target motion information to determine whether there is a motion error in the camera arm and/or the at least one operating arm.

In another aspect, a motion error detection method is provided. The motion error detection method for a surgical robot, wherein the surgical robot comprises a camera arm having a first image end instrument, at least one operating arm having more than one feature site, a second image end instrument having with respect to the camera arm and the at least one operating arm, and an input device configured for receiving at least one control command for controlling the camera arm and/or the at least one operating arm to move, comprises: obtaining at least one monitoring image of the camera arm and/or the at least one operating arm captured by the second image end instrument; identifying feature sites of the camera arm and/or the at least one operating arm of the at least one monitoring image as at least one first feature site; obtaining the at least one control command from the input device, and obtaining a kinematics model of the at least one operating arm according to the at least one control command; obtaining at least one second feature site matching the at least one first feature site in the kinematics model; and displaying the at least one second feature site on the display, comparing the at least one second feature site with the at least one first feature site to determine whether there is a motion error in the camera arm and/or the at least one operating arm.

In another aspect, a computer readable storage medium is provided. The computer readable storage medium comprises a storage medium storing a computer program configured to be loaded and executed by a processor to implement the motion error detection method.

In another aspect, a motion error risk detection device is provided. The motion error risk detection device for a surgical robot comprises a memory for storing a computer program, and a processor for loading and executing the computer program configured to be loaded and executed to implement the motion error detection method.

The present disclosure has the following beneficial effects:

By obtaining at least one operational image of a surgical field captured by the camera arm; identifying at least one of the feature sites of the at least one operating arm as at least one first feature site; obtaining the at least one control command from the input device, and obtaining a kinematics model of the at least one operating arm according to the at least one control command; obtaining at least one second feature site matching the at least one first feature site in the kinematics model; and comparing the relevant information of the at least one first feature site and the at least one second feature site, the present disclosure determines whether there is a motion error in the surgical robot.

### BRIEF DESCRIPTION OF THE DRAWINGS

FIG. 1 is a schematic diagram of a structure of a surgical robot according to an example embodiment of the present application;
FIG. 2 is a partial schematic diagram of a structure of a robot arm of the surgical robot shown in FIG. 1;
FIG. 3 is a partial schematic diagram of a structure of the operation state of an embodiment of the present application;
FIGS. 4 to 9 illustrate flow charts of a motion error detection method of a surgical robot according to embodiments of the present application;
FIG. 10 is a schematic diagram of a structure of an operating arm according to an embodiment of the present application;
FIGS. 11 to 15 illustrate flow charts of embodiments of a motion error detection method of a surgical robot;
FIGS. 16 to 20 are schematic diagrams of interfaces of different embodiments of the present application;
FIG. 21 illustrates a flow chart of an embodiment of a motion error detection method of a surgical robot;
FIG. 22 is a bottom view of the trocar according to an embodiment of the present application;
FIG. 23 illustrates a flow chart of an embody of a motion error detection method of a surgical robot;
FIG. 24 illustrates a flow chart of an embody of a motion error detection method of a surgical robot;
FIGS. 25 to 26 are schematic diagrams of structure of the robot arm according to another example embodiments of the present application;
FIG. 27 is a schematic diagram of a structure of a robot arm according to an example embodiment of the present application;
FIG. 28 is a schematic diagram of a structure of motion error detection of a surgical robot according to an example embodiment of the present application.

### DETAILED DESCRIPTION

For ease of understanding of the present application, the present application will be described more fully hereinafter with reference to the associated drawings. Preferred embodiments of the present application are set forth in the accompanying drawings. This application may, however, be embodied in many different forms and is not limited to the embodiments described herein. Rather, these embodiments are provided for the purpose of providing a more thorough and thorough understanding of the disclosure of the present application.

It should be noted that when an element is referred to as being " disposed on" another element, it may be directly on the other element or intervening elements may also be present. When an element is considered to be "connected" to another element, it may be directly connected to another element or intervening elements may be present at the same time. When an element is considered to be "coupled" to another element, it may be directly coupled to another element or intervening elements may be present at the same time. As used herein, the terms "vertical", "horizontal", "left", "right" and the like are intended for purpose of illustration only and are not intended to be limiting. As used herein, the terms "distal end" and "proximal end" are common terms in the art of interventional medical devices, where "distal end" refers to the end far away from the operator during the surgical procedure, and the "proximal end" refers to the end close to the operator during the surgical procedure.

Unless defined otherwise, all technical and scientific terms used herein have the same meaning as commonly understood by one of ordinary skill in the art. The terminology used herein is for the purpose of describing particular embodiments only and is not intended to be limiting of the invention. As used herein, the term "and/or" includes all combinations of one or more of the associated listed items.

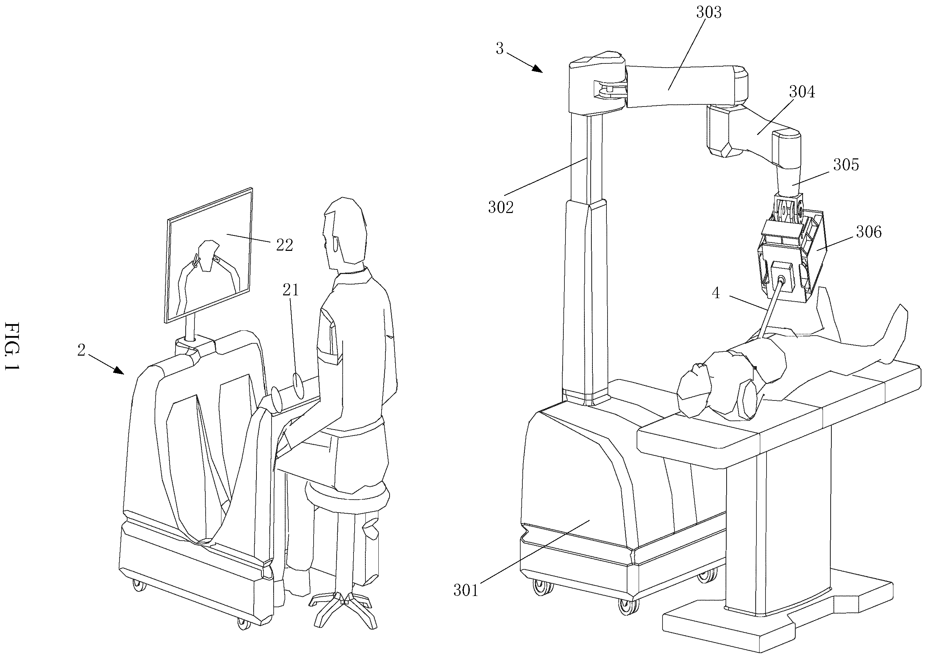

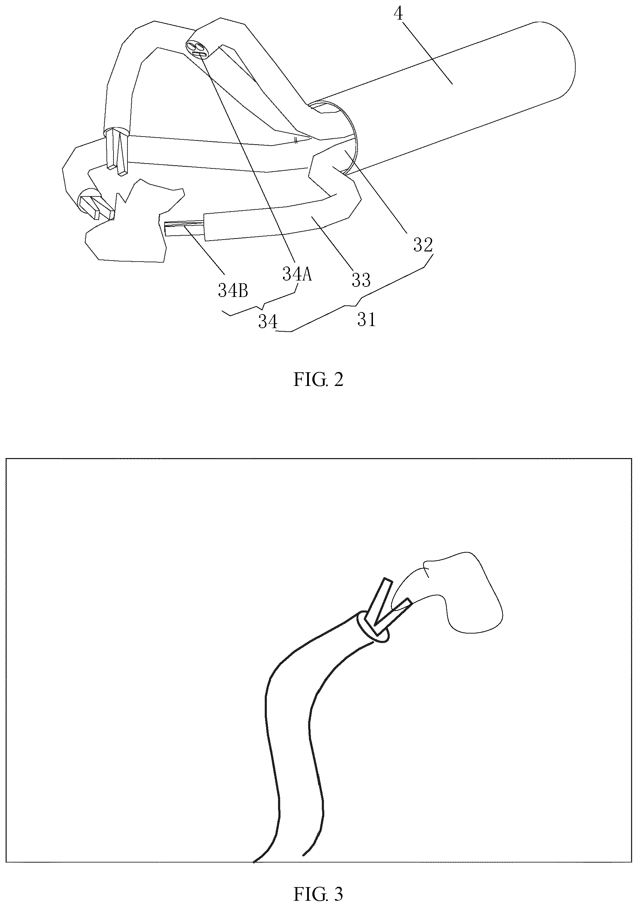

FIG. 1 is a schematic structural view of a surgical robot according to an embodiment of the present disclosure. FIG. 2 is a schematic partial view of the surgical robot of FIG. 1. FIG. 3 is a schematic partial view of the surgical robot of FIG. 1.

A surgical robot can include a master console 2 and a slave operating device 3. The master console 2 can have an input device 21 and a display 22. The doctor can give at least one control command to the slave operating device 3 by the input device 21, so that the slave operating device 3 can perform corresponding operation according to the control command(s) of the doctor's operating handle 21, and the surgical field can be observed on the display 22. The input device 21 can freely move and rotate, so that the doctor can have greater operating space, for example, the input device 21 can be a handle 21, and the handle 21 can be connected to the master console 2 by a connection wire. Alternatively, the handle 21 can be a mechanical handle, which is connected to the master console 2 by a rotatable linkage. The slave operating device 3 can comprise a robot arm 21 having a plurality of joint arms 301 ~ 306, the proximal joint arm 301 can be a base, and the remote joint arm 306 can be a power mechanism. The power mechanism 306 can be removably mountable on the robot arm(s) 31 and the power mechanism 306 can drive the robot arm(s) 31 to move. The robot arm(s) 31 can comprise a linkage 32, a connecting assembly 33 and an end instrument 34 in turn, wherein the periphery of the connecting assembly 33 can be covered with a sheath (sleeve), of course, it can also not be covered with a sheath, and the connecting assembly 33 can comprise a plurality of joints. The robot arm(s) 31 can adjust the position and/or orientation of the end instrument 34 by adjusting the joint; the end instrument 34 can include an image end instrument 34A and an operating end instrument 34B. In some embodiments, the input device 21 can also be a gesture tracking device, or an eye tracking device and so on, and the input device 21 can generate and input control command(s) by tracking hand movement(s) and/or eye movement(s).

The surgical robot can comprise a control device configured to be coupled to the joint arms 301 ~ 306, the at least one operating arm 31 and so on, so as to receive, process and send relevant command(s). The control device can be integrated into the main operation console 2 or the slave operating device 3; the control device can also be set independently of the master operation console 2 and the slave operation device 3, and the control device can be deployed locally or in the cloud. The control device can comprise at least one controller, such as one, two, or more.

The surgical robot illustrated in FIG. 1 is a single aperture surgical robot. In the single aperture surgical robot, robot arm(s) 31 can be inserted into a same aperture to enter the patient. More specifically, robot arm(s) can usually be inserted into the patient's body by connecting the power unit 306 and the trocar 4 through the aperture. The robot arm(s) 31 of a single aperture surgical robot generally can provide multiple degrees of freedom by multiple joints, such as three orientation degrees of freedom (pitch direction, yaw direction and rotation direction), and two positioning degrees of freedom (horizontal direction and vertical direction), and the power mechanism 306 can provide a positioning degree of freedom in the back and forth directions. In this way, in the conventional solution, the position of the main arm is kept unchanged, that is, the power mechanism 306 can be used as the dividing point, and the input device 21 can control the robot arm(s) 31 to move independently, so that the end instrument 34 at the distal end of the robot arm(s) 31 can move in 360° . Of course, generally, the robot arm(s) 31 can also have an opening and closing degree of freedom to complete the clamping motion.

Based on the type of the end instrument 34, the robot arm(s)31 can be divided into two categories. One is the camera arm with the image end instrument 34A, and the other is the operating arm with the operating end instrument 34B. Further, the camera arm can be further classified according to the type of the image end instrument 34A, and the operating arm can be further classified according to the type of the operating end instrument 34B, which will not be further described.

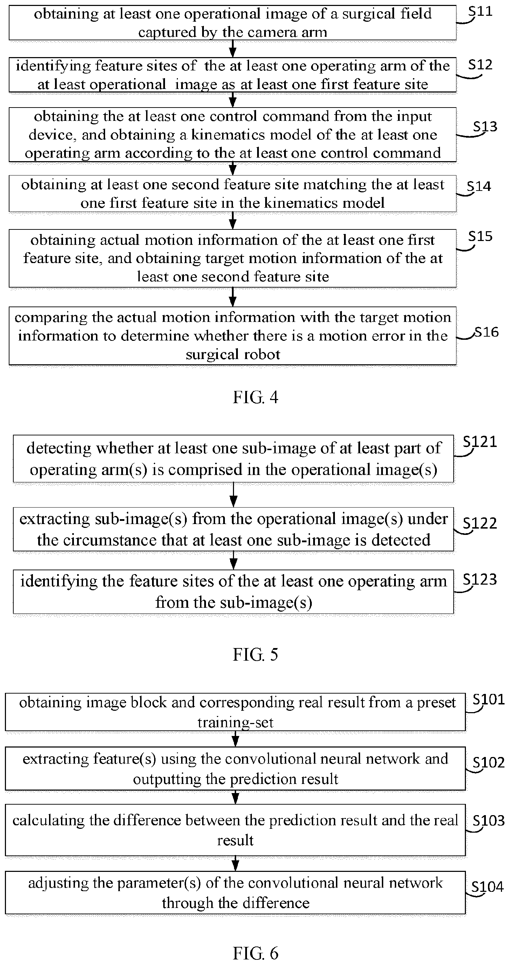

FIG.4 illustrates a flow chart of a motion error detection method of a surgical robot according to an embodiment of the present application. The detection method can be executed by the control device, and can begin at block S11.

At block S11, the method may include acquiring an operational image(s) of the surgical field captured by the camera arm.

The operational image(s) can be captured by the image end instrument of the camera arm, and the image end instrument cannot at least capture the image of its own light-enter surface (imaging surface).

At block S12, the method may include identifying feature sites of the at least one operating arm from the operational image(s), and using the identified feature sites as at least one first feature site;

The feature sites generally can refer to more than one of the linkage of the robot arm, joint, and the end instrument. For example, the feature sites are the same type of structure in the robot arm, for example, they are all linkages, joints, or end instruments. For another example, the feature sites are different types of structures in the robot arm, such as two or more types of linkages, joints, and end instruments, such as linkages and joints, linkages and end instruments, joints and end instruments, or linkages, joints, and end instruments.

According to block S12, the first feature site(s) can be all feature sites identified from the operational image(s). According to the identification result, there may be one first feature site, or there may be more than one first feature site.

At block S13, the method may include obtaining control command(s) from the input device, and obtaining a kinematics model of the at least one operating arm according to the control command(s).

At block S14, the method may include obtaining second feature site(s) matching the first feature site(s) in the kinematics model.

At block S14, the method may include matching the first feature site(s) actually appearing in the operational image(s) in the calculated kinematics model. For example, the operating arm(s) corresponding to the operational image(s) can comprise the first to sixth joints and an end instrument, assuming that the first feature sites refer to the end instrument, the first joint, the second joint and the third joint identified in the operational image(s), and then the corresponding second feature sites refer to the end device, the first joint, the second joint and the third joint in the kinematics model; for another example, assuming that the first feature sites refer to the end instrument, the first joint and the third joint identified in the operational image(s), and then the corresponding second feature sites refer to the end instrument, the first joint and the third joint in the kinematics model.

At block S15, the method may include obtaining actual motion information of the first feature site(s), and obtaining target motion information of the second feature site(s).

At block S16, the method may include comparing the actual motion information with the target motion information to determine whether there is a motion error in the surgical robot.

At blocks S11 to S16, only the identified feature sites are compared. That is, the feature sites found from the operational image are compared with the corresponding real feature sites in the robot arm, so it is not necessary to consider the situation where the comparing cannot be executed because of one or several feature sites without being identified.

At least when being compared, the actual motion information of the first feature site(s) and the target motion information of the second feature site(s) can be relative to the same reference coordinate system. For example, they all can be relative to the tool coordinate system of the image end instrument of the camera arm (referred to simply as under the viewpoint of the camera arm). By unifying the actual motion information and the target motion information in the reference coordinate system, it is helpful to compare the internal data or external presentation between them.

In one embodiment, at block S12, the method may begin at block S121.

At block S121, the method may include detecting whether at least one sub-image of at least part of operating arm(s) is comprised in the operational image(s).

The operational image(s) can refer to the image(s) that can be captured by the image end instrument of the camera arm, and the sub-image(s) can refer to the operational image(s) only associated with the at least one operating arm, that is, the sub-image(s) can be part of the operational image(s). The operational image(s) may comprise no sub-image, one sub-image, or multiple sub-images, and in the case of comprising sub-image(s), the sub-image(s) may be a global or local image(s) of the at least one operating arm.

The above detection can be performed by human, or be performed automatically by image recognition. At block S121, the detection can be performed automatically, such as operational image(s) may be input into a neural network, and whether at least one sub-image of the at least one operating arm is comprised in the operational image(s) is detected by the neural network.

Furthermore, under the circumstance that at least one sub-image is detected at block S 121, the method may proceed to block S122; under the circumstance that no sub-image is detected at block S121, the method may proceed to block S121.

At block S122, the method may include extracting sub-image(s) from the operational image(s).

For example, sub-image(s) may be extracted from the operational image(s) based on a threshold or an edge-based segmentation in the field of image processing.

At block S 123, the method may include identifying the feature sites of the at least one operating arm from the sub-image(s).

By pre-extracting sub-image(s), it is beneficial to improve the recognition efficiency of feature sites. The feature sites of each of the at least one sub-image belong to a same operating arm.

In one embodiment, at block S123, the method may include inputting the sub-image(s) into a neural network, and detecting the feature sites of the at least one operating arm based on the sub-image(s) through the neural network.

In one embodiment, at block S12, the method may include inputting the operational image(s) into a neural network, and detecting the feature sites of the at least one operating arm based on the operational image(s) through the neural network.

when at least one sub-image of at least part of multiple operating arms is comprised in the operational image(s), the identified feature sites may be assigned to the corresponding operating arm according to the difference in the contour(s) of the sub-image(s) or operating arm(s).

In one embodiment, at block S15, the method may include predicting the actual motion information of the first feature site(s) through the neural network, more specifically, a prediction value is predicted by the neural network, and the prediction value can be used as the corresponding actual motion information, under the circumstance that the accuracy rate of the prediction value reaches a threshold, for example, the accuracy rate threshold is set to be above 95%, e.g., 99%. The actual motion information and target motion information at block S15 can refer to position information and/or orientation information, or refer to velocity information and/or angular velocity information. The actual position information and/or orientation information can be obtained from one frame of operational image by the convolutional neural network; the actual velocity information and/or angular velocity information can be obtained from more than two frames of operational images by the convolutional neural network.

In another embodiment, at block S15, the method may include obtaining the actual motion information of the first feature site(s) under the viewpoint of image end instrument through the parallax principle, which requires the image end instrument of the camera arm to be an image end instrument with binocular vision (for example binocular endoscope). The actual position information and/or orientation information can be obtained from one frame of operational image through the parallax principle; the actual velocity information and/or angular velocity information can also be obtained from more than two frames of operational images through the parallax principle, typically, one frame of operational images is the initial image of each joint of the at least one operating arm in the zero position state, and the other frame of operational images is the current image of each joint of the at least one operating arm in the current state, so that the absolute joint variable(s) of the first feature site(s) can be obtained, which is beneficial to calculate the absolute actual velocity information and/or angular velocity information.

The above three neural networks can be independent of each other, or can be any combination of any two or more, such as three neural networks being a whole. The above three neural networks can include the neural network for detecting whether the at least one sub-image of the at least one operating arm is comprised in the operational image(s), the neural network for detecting the feature sites of the at least one operating arm in the operational image(s) or the sub-image(s), and the neural network for predicting the actual motion information of the first feature site(s).

Before using the neural network(s), the neural network(s) usually need to be trained. The neural network(s) may be convolutional neural network(s) (CNN). In brief, the convolutional neural network(s) may be trained according to a difference between a prediction result and a real result, wherein the prediction result can correspond to the feature image extracted from the preset training-set through the convolutional neural network, and the real result can correspond to the preset training-set.

The preset training-set is a collection of images of multiple types of robot arms available for surgical robots. For example, the robot arm is a camera arm. For another example, the robot arm can be at least one operating arm, and the operating arm(s) with the end instrument being a clamp, an electric knife, an electric hook, a scissors, or a stapler can be one type of the operating arm(s); and each type of the operating arm can be further subdivided according to the specific structure and specifications, for example, the operating arm(s) with 4 degrees of freedom (such as 3 orientation degrees of freedom and 1 opening and closing degree of freedom) can be one type of the operating arm(s), and the operating arm(s) with 7 degrees of freedom (for example omnidirectional degrees of freedom and 1 opening and closing degree of freedom) can be another type of the operating arm(s).

The relevant information of the image used as the input of the convolutional neural network in the preset training-set can be accurately calibrated according to the prediction result expected to be output by the convolutional neural network, which is beneficial to the training of the convolutional neural network.

For example, a convolutional neural network extracts a feature image from a given image and outputs a prediction result based on the feature image.

For example, whether the image comprises a robot arm can be determined. If the output prediction result is that the image does not comprise a robot arm, but the actual image comprises a robot arm, then the convolutional neural network is artificially told that the prediction result is wrong, and the contour and/or feature sites of the robot arm can be artificially calibrated; if the output prediction result is that the image comprises a robot arm, and the actual image comprises a robot arm, and then the convolutional neural network is artificially told that the prediction result is correct.

For another example, the feature sites of the robot arm in the image need to be detected. If the output prediction result, i.e. the identified feature sites is correct, the convolutional neural network is artificially told that the prediction result is correct; if the output prediction result, i.e. the identified feature sites is wrong, the convolutional neural network is artificially told that the prediction result is wrong, and the correct feature sites can be calibrated artificially.

For another example, joint variable(s) (or motion information such as position information and/or orientation information) corresponding to feature sites of the image need to be predicted. If the output prediction result, i.e. the joint variable(s) (or motion information) corresponding to the identified feature site is correct, then the convolutional neural network is artificially told that the prediction result is correct; if the output prediction result, i.e. the joint variable (or motion information) corresponding to the identified feature sites is wrong, then the convolutional neural network is artificially told that the prediction result is wrong, and the correct joint variable(s) (or motion information) corresponding to the feature sites can be calibrated artificially.

The prediction result of the convolutional neural network can also be the type of the robot arm, or it can be the type of the feature sites, or the position of the feature sites in the robot arm, etc.

The training the convolutional neural network can begin at block S101 illustrated in FIG. 6.

At block S101, the method may include obtaining image block and corresponding real result from a preset training-set.

At block S102, the method may include extracting feature(s) using the convolutional neural network and outputting the prediction result.

At block S103, the method may include calculating the difference between the prediction result and the real result.

At block S104, the method may include adjusting the parameter(s) of the convolutional neural network through the difference.

Blocks S101 to block S104 can be implemented until the output prediction result converges to the real result. The above block 5104 can be implemented through a backpropagation algorithm, but it is not limited thereto.

The feature image(s) can represent feature(s) extracted from an image. Each point in the feature image(s) corresponds to an image block whose center is the point in the image corresponding to the point in the feature image(s).

The backpropagation algorithm aims to describe a general method for training artificial neural networks in combination with optimization method (for example gradient descent method). This method is used to calculate the gradient of the loss function against all weights in the network. The above gradient may be fed back to the optimization method which may use the gradient to update the weight(s) to minimize the loss function.

The above prediction result may include the type of the robot arm and/or the position of the feature sites in the robot arm (for example the first few of joints or linkages), which is beneficial for the execution of the above block S15, and specifically beneficial for obtaining the second feature site(s) matching the first feature site(s) in the kinematics model quickly according to the type of the robot arm and/or the position of the feature sites in the robot arm.

In one embodiment, at block S15, the target motion information of the second feature(s) site may be calculated according to the kinematics model of the at least one operating arm. The target position information, target orientation information, target velocity information and/or target angular velocity information of the second feature site(s) can be calculated according to the kinematics model. Usually, only the type of the actual motion information is needed.

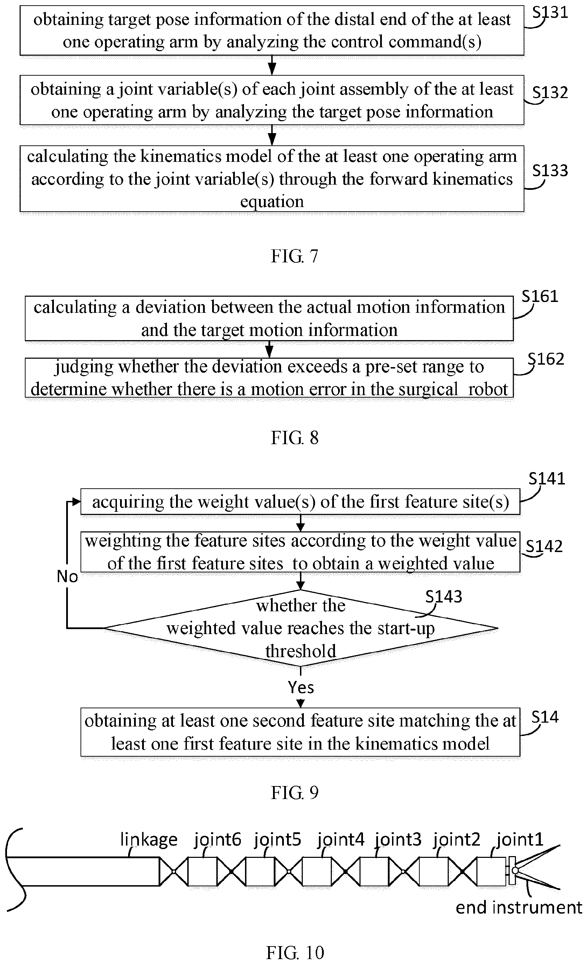

In one embodiment, the above block S13 can begin at block S131 illustrated in FIG. 7.

At block S131, the method may include obtaining target pose information of the distal end of the at least one operating arm by analyzing the control command(s).

At block S132, the method may include obtaining a joint variable(s) of each joint assembly of the at least one operating arm by analyzing the target pose information.

At block S133, the method may include calculating the kinematics model of the at least one operating arm according to the joint variable(s) through the forward kinematics equation.

In one embodiment, as shown in FIG. 8, at the above block S16, the method may include:

At block S161, the method may include calculating a deviation between the actual motion information and the target motion information.

At block S162, the method may include judging whether the deviation exceeds a pre-set range.

There is a motion error in the surgical robot under the circumstance that the deviation exceeds the preset range; there is no motion error in the surgical robot under the circumstance that the deviation does not exceed the preset range.

In one embodiment, the method further may comprise generating at least one motion error warning message after block S16, at which the method can determine there is a motion error in the surgical robot.

The warning message(s) can be displayed on a display. For example, at least one warning identification corresponding to the warning message(s) can be generated in the sub-image(s) and displayed on the display. In one embodiment, the at least one warning identification may be contour line(s), and when the at least one warning identification is generated in the sub-image(s), the contour line(s) can be correspondingly generated on the contour(s) of the sub-image(s). Usually, the contour line(s) can be prominently displayed on the contour of the sub-image(s), for example, using a different color and/or line types and/or flashing.

The surgical robot can comprise an audio device, such as a speaker, coupled to the control device. Auditory feedback corresponding to the warning message(s) can be generated using the audio device. The audio device can be set in the master operation console, and the audio device can also be set in the slave operating device. Certainly, the audio device can be set outside of the surgical robot.

The surgical robot can comprise a haptic device coupled to the control device. Haptic feedback corresponding to the warning message(s) can be generated using the haptic device.

For example, the haptic device is a vibration device, and the haptic feedback is in the form of vibration. Typically, the vibration intensity can be adaptively adjusted according to the degree of the deviation exceeding the predetermined range, for example in a positively correlated way, and the deviation is between the actual motion information and the target motion information. The higher the degree of the deviation exceeds the pre-set range ; , the higher the vibration intensity. The vibrating device can be set in the master operation console that can be in contact with the doctor's body, for example the armrest or the foot pedal of the master operation console.

For another example, the haptic device can be a resistance device, and the haptic feedback can be in the form of a resistance force. Typically, the resistance force can be adaptively adjusted according to the degree of the deviation exceeding the pre-set range, for example in a positively correlated way, and the deviation is between the actual motion information and the target motion information. The higher the degree of the deviation exceeds the pre-set range, the greater the resistance force.

In one embodiment, after block S16, at which the method determines that there is a motion error in the surgical robot, the method may further include generating a control command forbidding a movement of the at least one operating arm to prevent the at least one operating arm from being controlled to move; alternatively, generating a control command forbidding an inputting of the input device to prevent an input from the input device, which is equivalent to cutting off the input of the control command(s). In another embodiment, the input device may be a mechanical handle. Each joint of the mechanical handle can have a motor for driving the movement of the corresponding joint(s). After block S16, at which the method determines that there is a motion error in the surgical robot, the method may further include generating a control command of increasing the torsion and/or torque of each of the motor(s) to form a resistance force which can prevent the movement of the mechanical handle, so that preventing the mechanical handle from being moved by the force from the doctor, which can cut off the input of the control command(s). By inhibiting the movement of the at least one operating arm, or cutting off the input of the input device, possible surgical risk due to undesired movement of the at least one operating arm can be prevented.

In one embodiment, before block S14, the method may begin at block S141 illustrated in FIG. 9.

At block S141, the method may include acquiring the weight value(s) of the first feature site(s) .

The weight value of each feature site may be the same or different. For example, when the feature sites are all joints, the joints at the proximal end of the end instrument can be set a relatively higher weight value, and other feature sites can be set a relatively lower weight value, because usually the motion state of the joints at the proximal end of the end instrument can best directly reflect the motion state of the entire at least one operating arm.

At block S142, the method may include weighting the feature sites according to the weight value of the first feature sites to obtain a weighted value.

At block S143, the method may include judging whether the weighted value reaches the start-up threshold.

The method proceeds to block S14 under the circumstance that the weighted value is equal or greater than the start-up threshold; The method proceeds to block S141 under the circumstance that the weighted value does not reach the start-up threshold.

For example, an operating arm comprising 1 linkage and 6 joints together has more than 3 degrees of freedom illustrated in FIG. 10. The linkage may be equipped with joints 1-6 in turn, joint 1 may be equipped with an end instrument, and the linkage can drive joints 1-6 and the end instrument to rotate; the group of joints 1-3 and the group of joints 4-6 can be controlled independently, and both groups have two yaw degrees of freedom, and then by controlling the two groups to cooperate, 4 degrees of freedom can be realized. Combining the degrees of freedom of the linkage and the two groups of joints the method can realize 6 spatial degrees of freedom, and can further include the opening and closing degrees of freedom of the end instrument. For example, weight values of joints 1 to 6 are [0.4, 0.2, 0.15, 0.1, 0.1, 0.05] . For example, the start-up threshold is 0.4. Under the circumstance that only joint 1 is recognized, because the weighted value just reaches the start-up threshold of 0.4, the method can proceed to block S 14; under the circumstance that joint 1 is not recognized due to occlusion, etc., and joints 2-4 are recognized, and the sum of weighted values is 0.45, because the weighted value reaches the start-up threshold of 0.4, the method proceeds to block S14; under the circumstance that only one of joints 2-6 is recognized, or the sum of the weighted value(s) does not reach the start-up threshold, the method cannot proceed to block S14. Of course, the start-up threshold can also be increased, so that enough feature sites can be recognized before proceeding to block S14.

By the above block S11 to S16, it is possible to objectively detect whether there is a motion error in the surgical robot through the basic configuration of the surgical robot. In addition, the operation risk worsening can be avoided by the warning message(s) of the movement error and/or limiting further occurrence of the movement error.

The above embodiments can be applicable to the case of one or more at least one operating arms, even if adjacent operating arms are occluded. It is only necessary to accurately assign the identified first feature site(s) to the corresponding operating arm, and the assigning method is simple. For example, the first feature site(s) can be assigned to the corresponding operating arm according to the similarities and differences of the contour(s). The first feature site(s) can also be assigned to the corresponding operating arm according to the similarities and differences of the attribute of the first feature site(s), which is not repeated here.

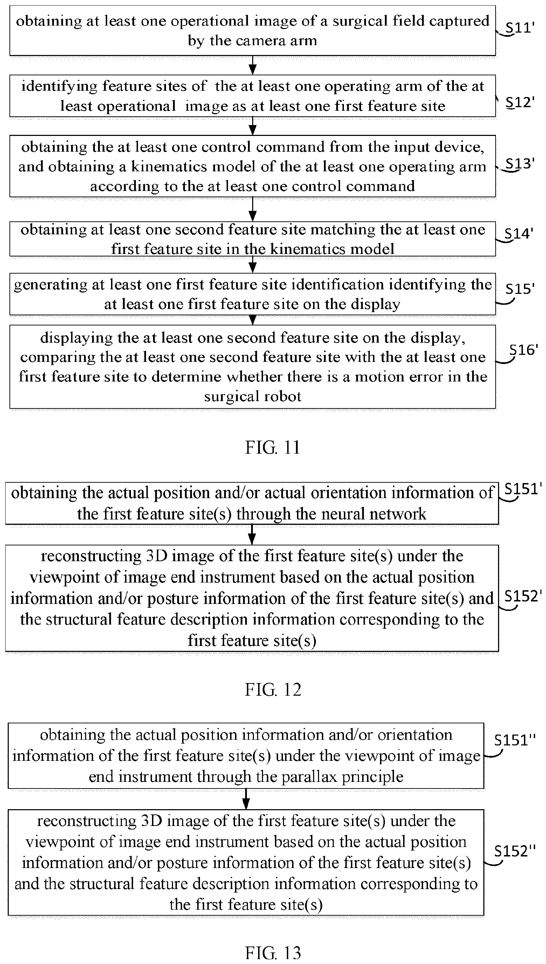

In one embodiment, FIG. 11 illustrates another method for detecting the motion error of a surgical robot. The method can begin at block S11'.

At block S11', the method may include obtaining an operational image(s) of the surgical field captured by the camera arm.

At block S12', the method may include identifying feature sites of the at least one operating arm(s) from the operational image(s), and using the identified feature sites as first feature site(s).

At block S13', the method may include obtaining a control command from the input device, and obtaining a kinematics model of the at least one operating arm according to the control command.

At block S14', the method may include obtaining a second feature site(s) matching the first feature site(s) in the kinematics model.

At block S15', the method may include generating at least one first feature site identification on the display.

Block S15' can be omitted under the circumstance that the at least one first feature site itself is obvious and easy to be compared with the second feature site(s). For example, the at least one first feature site itself has feature point(s) characterizing the first feature site(s), and the feature point(s) can be clearly visible. For another example, the at least one first feature site itself is clearly visible and comprises a clear contour. An identification may be generated at the first feature site(s) for more enhanced comparison even if the at least one first feature site is already clearly visible.

The first feature site identification(s) may comprise at least one of pattern and color.

At block S16', the method may include displaying at least the second feature site(s) on the display, so that the second feature site(s) and the first feature site(s) can be compared to determine whether there is a motion error in the surgical robot.

Blocks S11' to S14' can be implemented in a manner similar to or same as that of block S11 to S14. Repetitive descriptions are omitted to avoid redundancy.

At blocks S15' to S16', the method may include observing the contents on the display by human eyes, specifically determining whether there is a movement error of the surgical robot by comparing the first feature site(s) and the second feature site(s). This method is suitable for the situation where the deviation between the actual motion state and the target motion state of the operating arm(s) is large, at least visible to the naked eye. This method is suitable for experienced or trained doctors, who can accurately determine whether there is a motion error in the surgical robot even if the deviation is small.

In one embodiment, at block S15', the method may include generating at least one the first feature site identification in sub-image(s) and displaying the at least one first feature site identification on the display for the purpose of observation. For example, the display has at least a first display window, and the operational image(s), sub-image(s), and the identification in sub-image(s) can be displayed on the first display window. For another example, the display can have a first display window and a second display window, and the first display window can be used as the main display window. The operational image(s) can be displayed on the first display window. The identification(s) in sub-image(s) cannot be displayed on the first display window, which can avoid disturbing the doctor. The identification(s) in sub-image(s) can be displayed on the second display window. The zoomed operational image(s) can be displayed on the second display window. The separately extracted and zoomed sub-image(s) can be separately displayed on the second display window. The identification(s) can be displayed in the sub-image(s) of the zoomed operational image(s), and the identification(s) can be displayed in the separate sub-image(s).

In one embodiment, at block S15', the method may include generating the identification at a position corresponding to the first feature site(s) in three-dimension (3D) image. The 3D image can be reconstructed based on the actual motion information, e.g., actual position information of the first feature site(s). The 3D image can be displayed on the display for comparison. For example, the first feature site(s) can comprise the first joint, the second joint and the first linkage between the first joint and the second joint, then the corresponding 3D image can comprise the first joint, the second joint and the first linkage. The reconstructed 3D image corresponding to the first feature site(s) may reflect the structure of the part, e.g., the back, and the part is beyond the sight of the image end instrument of the camera arm.

In one embodiment, the reconstruction may include blocks S151'and S152' illustrated in FIG. 12.

At block S151', the method may include obtaining the actual position and/or actual orientation information of the first feature site(s) through the neural network.

The training method and principle of the neural network are basically the same as those described in the foregoing embodiments.

At block S152', combining the actual position and/or actual posture information of the first feature site(s), and the structural feature description information corresponding to the first feature site(s), to reconstruct the three-dimensional view of the first feature site(s) under the viewpoint of the image terminal device of the camera arm image.

The viewpoint essentially can refer to the coordinate system. Both the viewpoint of the image end instrument and the camera arm can refer to the tool coordinate system of the image end instrument.

In another embodiment, as shown in FIG. 13, the above-mentioned reconstruction may include:
At block S151", the method may include obtaining the actual position information and/or orientation information of the first feature site(s) under the viewpoint of image end instrument through the parallax principle.

At block S151", the image end instrument of the camera arm can be an image end instrument with binocular vision, such as binocular endoscope.

At block S152", the method may include reconstructing 3D image of the first feature site(s) under the viewpoint of image end instrument based on the actual position information and/or posture information of the first feature site(s) and the structural feature description information corresponding to the first feature site(s).

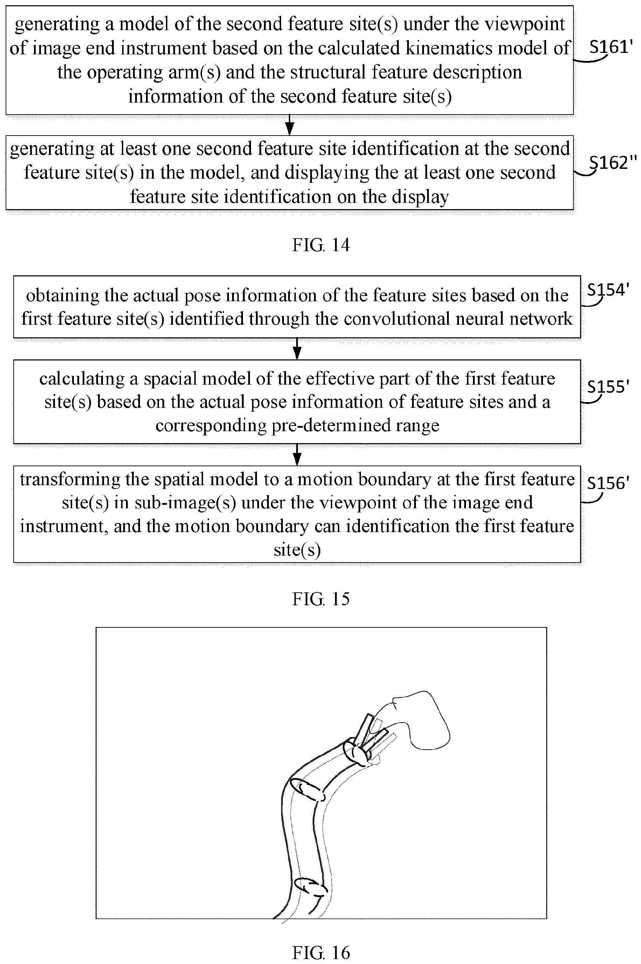

In one embodiment, at block S16', the method may begin at block S161" illustrated in FIG. 14.

At block S161", the method may include generating a model of the second feature site(s) under the viewpoint of image end instrument based on the calculated kinematics model of the operating arm(s) and the structural feature description information of the second feature site(s).

The model of the second feature site(s) can be a computer model or a projection model. The former can more accurately reflect the structure of the second feature site(s), and the latter can also basically accurately reflect the structure of the second feature site(s).

At block S162", the method may include generating at least one second feature site identification at the second feature site(s) in the model, and displaying the at least one second feature site identification on the display.

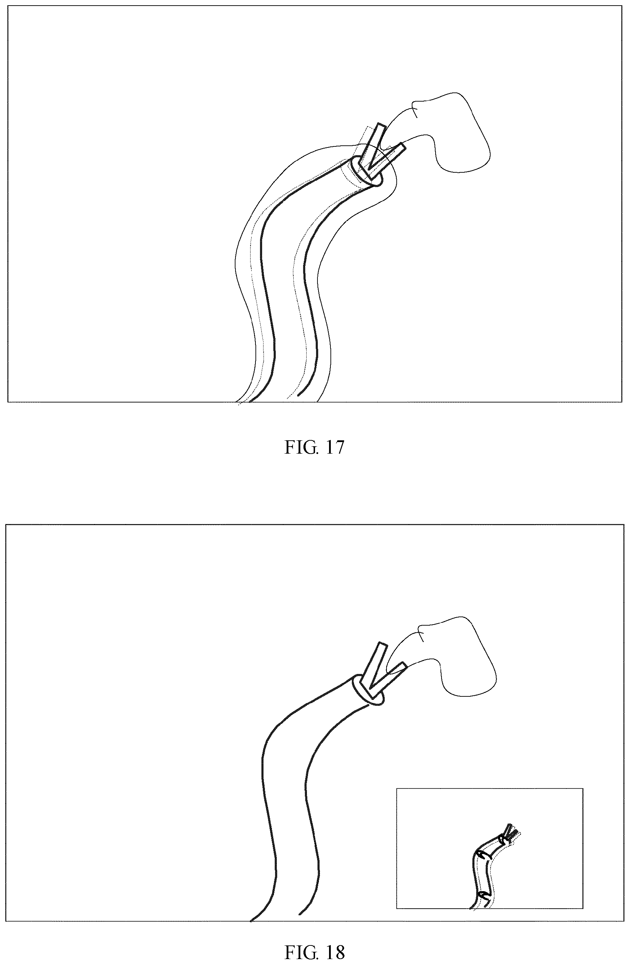

Similarly, in order not to avoid disturbing the doctor's surgical operation by the operational image(s), the display can have a first display window and a second display window, and the operational image(s) can be displayed in the first display window. The reconstructed 3D image corresponding to the first feature site(s) or the model of the second feature site can be displayed in the second display window for comparison, and these 3D image(s) or model can usually have been zoomed illustrated in FIGS. 18 to 20.

In order to more obviously and conveniently judge whether there is a motion error in the surgical robot for doctors, in one embodiment, at block S15', the method may begin at block S154' illustrated in FIG. 15.

At block S154', the method may include obtaining the actual pose information of the feature sites based on the first feature site(s). The first feature site(s) can be predicted through the convolutional neural network.

At block S155', the method may include calculating a spacial model of the effective part of the first feature site(s) based on the actual pose information of feature sites and a corresponding pre-determined range.

At block S156', the method may include transforming the spatial model to a motion boundary at the first feature site(s) in sub-image(s) under the viewpoint of the image end instrument, and the motion boundary can identification the first feature site(s). Repetitive descriptions of reconstruction are omitted to avoid redundancy.

At block S156', the 3D image(s) can comprise the motion boundary. The repetitive descriptions of reconstruction of the 3D image(s) are omitted to avoid redundancy.

At block S154'~S156', the method may include enlarging the motion boundary of the first feature site(s) based on the pre-determined range of the first feature site(s). When the doctor judges whether there is a motion error in the surgical robot by observation, the doctor can judge whether there is a motion error in the surgical robot only by judging whether the corresponding second feature site(s) is within the motion boundary or the degree within the motion boundary.

The motion boundary can be in the form of contour(s). The actual contour(s) (i.e., edge) of the first feature site(s) may have been substantially enlarged.

Under the circumstance that the first feature site(s) is spaced part of the at least one operating arm, for example, only joints, or only linkages, the contours of the first feature site(s) are basically independent of each other, and there may also be some intersections, but generally it can be judged that they are independent of each other illustrated in FIG. 16. Under the circumstance that the spaced first feature sites comprise independent points, the contour corresponding to each point is basically independent of each other.

Under the circumstance that the at least one first feature site(s) is a continuous part, such as joint(s) and linkage(s) connected with joint(s), the contour of the first feature site(s) can join together. Under the circumstance that the first feature site(s) is a continuous line, the contour of the outline is a whole.

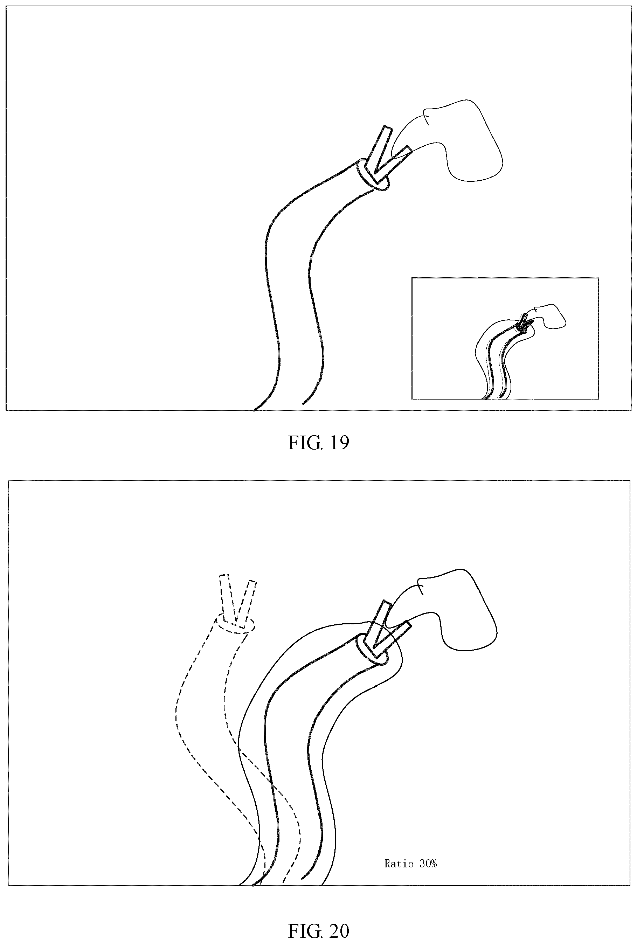

For example, under the circumstance that the at least one second feature site is not within the motion boundary of the first feature site(s), there is a motion error in the surgical robot. More complicatedly, part of the second feature site(s) is within the motion boundary of the first feature site(s), and the doctor can judge whether there is a motion error in the surgical robot according to the approximate degree of within the motion boundary. For example, because the at least one second feature site is basically or completely within the motion boundary of the first feature site(s) illustrated in FIGS. 16 to 19, the doctor can judge that there is no motion error in the surgical robot. However, because most of the at least one second feature site is not within the motion boundary of the first feature site(s) illustrated in FIG. 20, the doctor can judge that there is a motion error in the surgical robot.

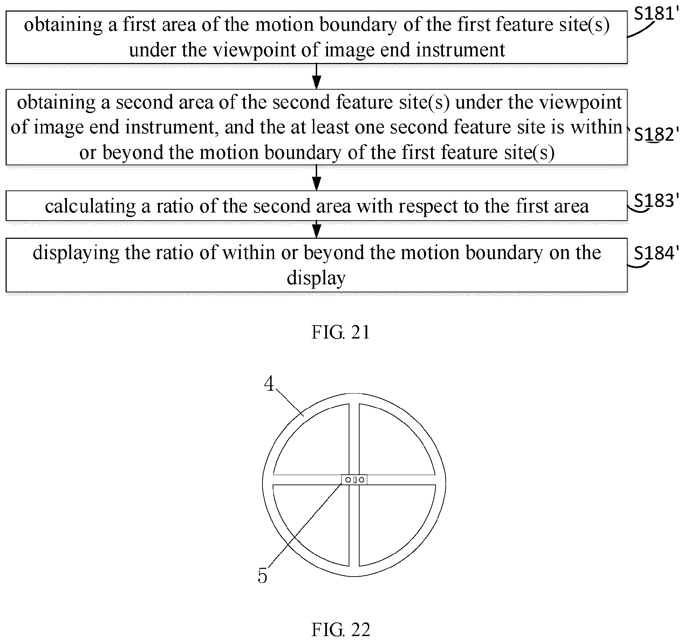

In one embodiment, part of the second feature site(s) is within the motion boundary of the first feature site(s) illustrated in FIG. 21, the methods may begin at block S181' in order to help the doctor to judge whether there is a motion error.

At block S181', the method may include obtaining a first area of the motion boundary of the first feature site(s) under the viewpoint of image end instrument.

At block S182', the method may include obtaining a second area of the second feature site(s) under the viewpoint of image end instrument, and the at least one second feature site is within or beyond the motion boundary of the first feature site(s).

At block S183', the method may include calculating a ratio of the second area with respect to the first area.

At block S184', the method may include displaying the ratio of within or beyond the motion boundary on the display.

By displaying this ratio on the display, it is helpful for the doctor to judge whether there is a motion error in the surgical robot. For example, the doctor knows there is no motion error in the surgical robot under the circumstance that the ratio of the second feature site(s) being within the motion boundary of the first feature site(s) exceeds 60%, and the ratio on the display is 30% illustrated in FIG. 20, so the doctor can quickly judge that there is no motion error in the surgical robot.

At the above block S15' ~ S16', under the circumstance that the doctor judges that there is a motion error in the surgical robot, the doctor can give a control command that can prohibit the movement of the at least one operating arm and camera arm; alternatively, the doctor can give a control command that can prohibit the input device from inputting control command(s).

Blocks S15' to S16' can also be implemented for waning in the embodies after block S14, and repetitive descriptions are omitted to avoid redundancy.

In the above embodiment, the camera arm equipped with image end instrument can be used to accurately detect whether there is a motion error in the entire surgical robot system, which is helpful for doctors to use the surgical robot safely and reliably, and the camera arm is the least necessary part of the surgical robot.

In some embodiments, and whether there is a motion error in one or both of operating arm(s) and the image arm in the entire surgical robot system can be accurately detected. An image end instrument can be additionally configured, and the additional image end instrument can be independent of the surgical robot, or can be integrated in the surgical robot, and the additional image end instrument can be independent of the camera arm and the at least one operating arm. It is at least promised that the pose information of the additional image end instrument is accurate and reliable, for example, the pose information of the additional image end instrument is fixed, and/or the pose information of the additional image end instrument can be accurately detected or calculated even if the pose information changes and so on. For understanding and distinction, the foregoing image end instrument in the camera arm can be the first image end instrument, and the additional image end instrument here can be the second image end instrument.

In one embodiment, the pose information of the second image end instrument can be fixed during the operation. For example, for a single- aperture surgical robot, the second image end instrument 5 can be set at the distal end of the trocar 4, and usually be inserted into the patient's body through the trocar 4 without other additional component or aperture illustrated in FIG. 22. For another example, the second image end instrument 5 can be set at the distal end of the straight rod, and the proximal end of the straight rod can be relatively fixedly set at the distal end of a mechanical arm, for example a power mechanism, which is equivalent to an image arm without degree of freedom or with at least one degree of freedom but being accurately and reliably detected or calculated, and the pose information of the second image end instrument can also be accurately and reliably detected or calculated. For another example, for a multi- aperture surgical robot, the second image end instrument may be set on the main arm of the multi-aperture surgical robot, for example the second image end instrument may be set on an orientation platform at the distal end of the main arm. As long as the minimum requirements of the second image end instrument are met, there are no excessive restrictions here.

The first image end instrument of the camera arm that can be controlled to move is mainly used to capture images of the surgical field to provide operational image(s), and operational image(s) can be helpful for the doctor to perform the operation. At least the light-enter surface (imaging surface) of the first image end instrument cannot be observed from the operational image(s). Some joints or rods of the camera arm can be observed by the first image end instrument, because the camera arm may be with necessary degree(s) of freedom. The second image end instrument can mainly be used to capture image(s) of the camera arm and the operating arm(s) for providing monitoring image(s), and at least part of the camera arm can be observed from the monitoring image(s). The monitoring image(s) may or not be displayed on the display.

For example, monitoring image(s) of the camera arm and the at least one operating arm can be captured by the second image end instrument may be obtained, and be displayed on the display. From the monitoring image(s), the doctor can observe the collision(s) between the operating arms or between the operating arm(s) and the camera arm, and the collision(s) cannot be observed by the first end instrument, especially being helpful to observe the collision(s) at the proximal part.

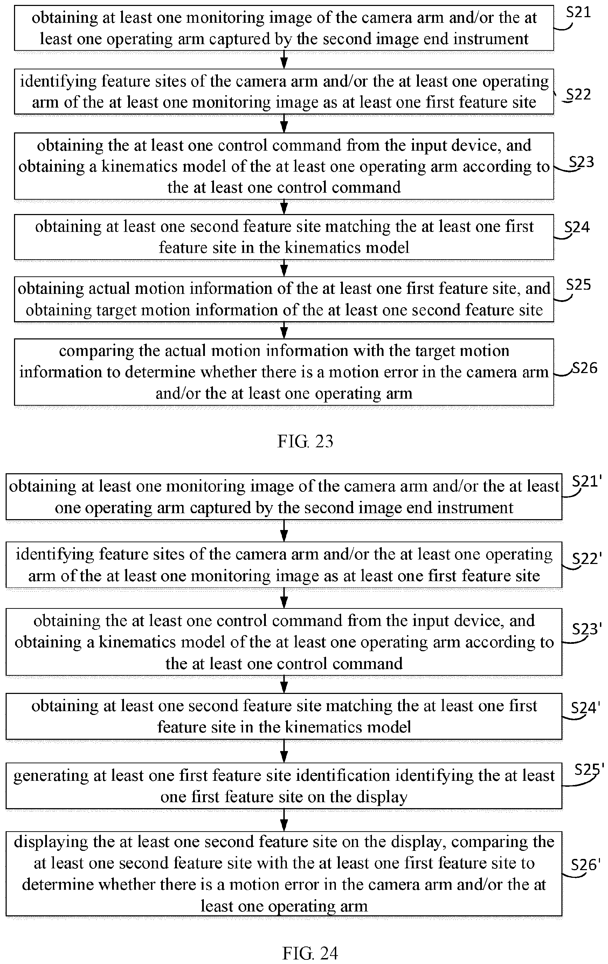

For example, monitoring image(s) of the camera arm and the operating arm(s) captured by the second image end instrument may be obtained and be used to determine whether there is a motion error in the surgical robot. Monitoring image(s) may be further used to determine whether there is a motion error in one or both of the operating arm(s) and the image arm in the surgical robot. In one embodiment, another method for detecting a motion error in a surgical robot is provided illustrated in FIG. 23. This detection method may begin at the block S21.

At block S21, the method may include obtaining the monitoring image(s) of the camera arm and/or the operating arm(s) captured by the second image end instrument.

At block S22, the method may include identifying the feature sites of the camera arm and/or the operating arm(s) from the monitoring image(s), and using the identified feature sites as the first feature site(s).

The identified feature sites can also be used as the first feature site(s). The feature sites of the camera arm can be defined correspondingly with reference to the definition of the feature sites of the operating arm(s) in the foregoing embodiments. For example, for the camera arm, the feature sites are the same type of structure in the camera arm, for example, they are all linkages, joints, or end instruments. For another example, the feature sites are different types of structures in the camera arm, such as two or more of linkages, joints, and end instruments, such as linkages and joints, or linkages and end instruments, or joints and end instruments, or linkages, joints, and end instruments.

At block S23, the method may include obtaining the control command(s) from the input device, and obtaining the kinematics model of the camera arm and/or the operating arm(s) according to the control command(s).

At block S24, the method may include obtaining a second feature site(s) matching the first feature site(s) in the kinematics model.

At block S25, the method may include obtaining the actual motion information of the first feature site(s) and the target motion information of the second feature site(s).

At block S26, the method may include comparing the actual motion information with the target motion information to determine whether there is a motion error in the camera arm and/or the operating arm(s).

At the above block S21 to S26, whether the camera arm or the at least one operating arm has a motion error can be detected. In addition, the operation risk worsening can be avoided by at least one motion error warning message of the movement error and/or limiting further occurrence of the movement error.

When avoiding the worsening of surgical risk by limiting further occurrence of the movement error, for example, after determining that there is a motion error in the camera arm and/or the operating arm(s) at block S26, the method may further include generating a control command forbidding a movement of the camera arm and/or the operating arm(s) to prevent the at least one operating arm from being controlled to move; alternatively, generating a control command forbidding an inputting of the input device to forbid the input of the control command to the input device. At least the movement of the arm with the movement error is limited.

Blocks S21 to S26 can be implemented in a manner similar to or same as that of blocks S11 to S16 and blocks S11' to S16', especially blocks S21 to S26 can be directly implemented in a manner same as that of blocks S11 to S16. For example, sub-image(s) of the camera arm and/or operating arm(s) can also be extracted from the monitoring image(s), and then the corresponding feature sites can be quickly identified from the sub-image(s). Repetitive descriptions are omitted to avoid redundancy.

The embodiments applicable to the operating arm(s) are also applicable to the camera arm, so special descriptions are omitted.

In one embodiment, another method for detecting a motion error in a surgical robot is provided. The method may begin at block S21' illustrated in FIG. 24.

At block S21', the method may include obtaining the monitoring image(s) of the camera arm and/or the operating arm(s) captured by the second image end instrument.

At block S22', the method may include identifying the feature sites of the camera arm and/or the operating arm(s) from the sub-image(s).

The identified feature sites can also be used as the first feature site(s).

At block S23', the method may include obtaining the control command(s) from the input device, and obtaining the kinematics model of the camera arm and/or the operating arm(s) according to the control command(s).

At block S24', the method may include obtaining a second feature site(s) matching the first feature site(s) in the kinematics model.

At block, the method may include generating the first feature site identification(s) on the display.

Block S25' can be omitted under the circumstance that the first feature site(s) is obvious and easy to be compared with the second feature site(s).

At block S26', the method may include displaying at least the second feature site(s) on the display, so that the second feature site(s) and the first feature site(s) can be compared to determine whether there is a motion error in the surgical robot.

Blocks S21' to S26' can be implemented in a manner similar to or same as that of blocks S11 to S16 and blocks S11' to S16', especially blocks S21' to S26' can be directly implemented in a manner same as that of blocks S11' to S16'.

The embodiments applicable to the operating arm(s) are also applicable to the camera arm, so special descriptions are omitted.

The motion error detection method of the above embodiments can be applied to any of the preoperative, intraoperative, and postoperative to verify the reliability of the surgical robot system.

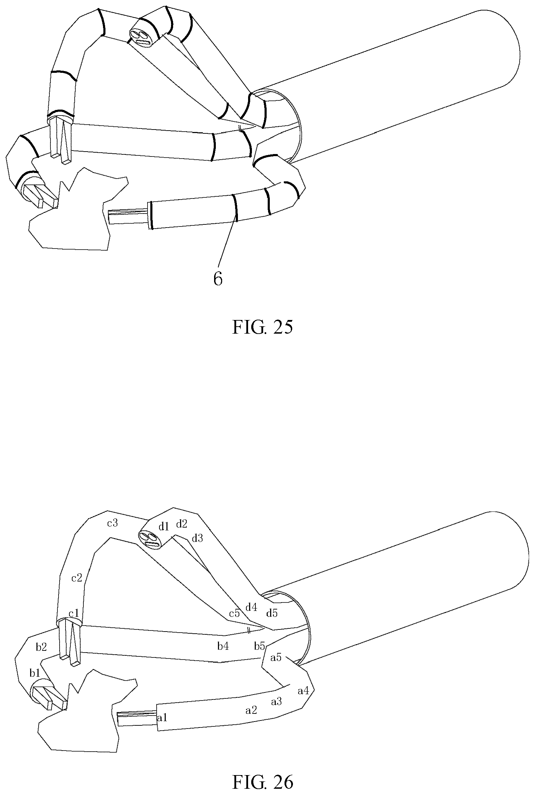

In the above embodiments, there may be some easily identified feature point(s) in the feature sites of the operating arm(s) and the camera arm. In order to more effectively identify the feature sites of the operating arm(s) and the camera arm in the sub-image(s), one or more feature points can be set for each of these feature sites to characterize these feature sites. The feature point(s) can be equivalent to a prominent identification. These feature point(s) can be embodied in various ways illustrated in FIGS. 25 and 26.

For example, the feature point(s) can comprise plane pattern, stereo geometry, or color, especially some colors that are significantly different from the color of the feature sites body. The feature point(s) can comprise at least two of plane pattern, stereo geometry, and color.

For example, when the feature point(s) can comprise a plane figure, it can comprise one or more points on the plane, or it can comprise one or more lines on the plane (illustrated in FIG. 25), and it can also comprise one or more planes on the plane. It can also comprise at least two of point, line, and plane. In one embodiment, the feature point(s) can comprise a graphic code, where the graphic code can have a pattern, and the correlation between the pattern and the feature sites can be established, so that the joint variable(s) of the associated feature sites can be obtained by calculating the zoomed state and/or the rotation state of the pattern under the viewpoint of the image end instrument. The graphic code may comprise a two-dimensional code or a barcode. For example, feature point(s) can be expressed as letters and/or numbers formed by multiple points or lines, for example the same letters and/or numbers can be set around the same feature site to facilitate identification of the same feature site, and different letters and/or numbers are set around the different feature sites to distinguish these different feature sites illustrated in FIG. 26.

For example, the feature point(s) can comprise a three-dimensional configuration, it can comprise one or more convex or concave points, lines, or planes. It can also comprise at least two of convex or concave point, line and plane. In order to increase the significance of feature point(s), special shapes can also be constructed, such as small cylinders, spheres, and pyramids and so on.

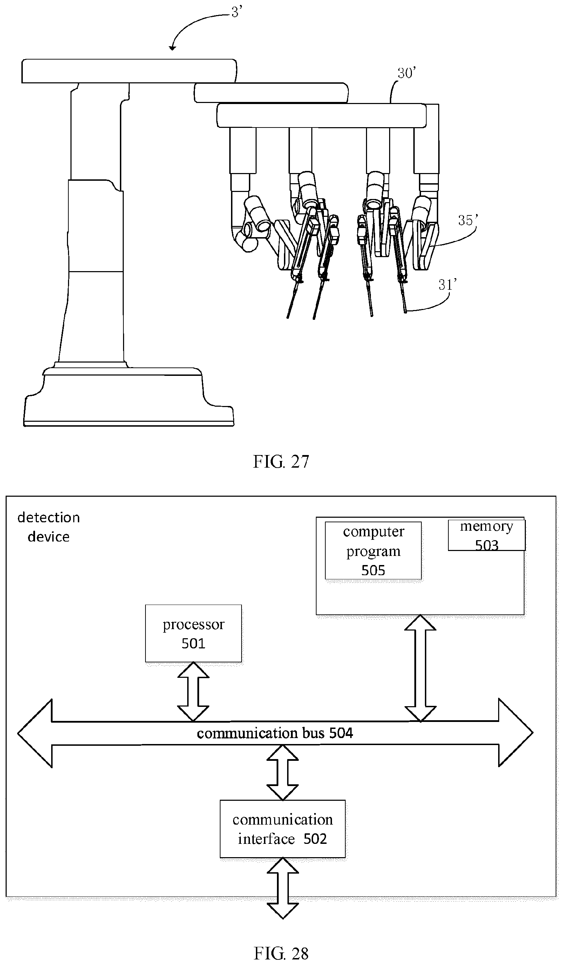

FIG. 27 is a schematic diagram of a structure of a multi-aperture robot arm. The slave operating device 3' of the multi-aperture surgical robot can comprise a main arm 30' and parallel manipulator(s) 35' at the distal end of the main arm 30', and each manipulator 35' can be equipped with a robot arm(s) 31'. The above embodiments are also applicable to the multi-aperture surgical robot, even though the robot arm(s) 31' of the multi-aperture surgical robot is mainly used to orient the distal end instrument, and the manipulation(s) 35' of the at least one operating arm 31' is mainly used to orient the distal end instrument. In the above applicable embodiments, the manipulator(s) 35' and the robot arm(s) 31' in the multi-hole surgical robot can be regarded as a whole being equivalent to the robot arm(s) 31 in the single-aperture surgical robot.

In an embodiment, a computer readable storage medium is provided, and a computer readable storage medium stores a computer program, which is configured to be loaded and executed by a processor to perform the detection method described in any of the above embodiments.

In an embodiment, the detection method of the above surgical robot is generally configured to be implemented in the control device of the surgical robot, the detection device comprises a memory for storing a computer program; and a processor for loading and executing the computer program; the computer program is configured to be loaded and executed by a processor to implement the detection method described in any of the above embodiments.

In some embodiments, the control command input by the input device may also refer to an instruction to control the opening and closing of the end instrument.

In some embodiments, the motion error detection device may comprise a processor 501, a communication interface 502, a memory 503, and a communication bus 504 illustrated in FIG. 28.

The processor 501, the communication interface 502, and the memory 503 can communicate with each other by the communication bus 504.

The communication interface 502 is configured to communicate with network element(s), for example various sensor(s) or motor(s) or solenoid valve(s) or other client(s) or server(s).

The processor 501 is configured for loading and executing the computer program 505 to carry out the detection method described in any of the above embodiments.

The program 505 may include program codes including computer operation instructions.

The processor 505 may be a central processing unit (CPU), a application specific integrated circuit (ASIC), one or more integrated circuits configured to implement one or more of the embodiments of the present application, or a graphics processing unit (GPU). The detection device can comprise at least one processor. Each processor can be the same type of processors, such as one or more CPUS, or one or more GPUS; each processor can also be different types of processors, such as one or more CPUS and One or more GPUS.

The memory 503 is configured to store the program 505. The memory 503 may be a high-speed read-only memory, and may also be a non-volatile memory, such as at least one disk memory.

The computer program 505 is loaded by the processor and carried out by the following steps: obtaining at least one operational image of a surgical field captured by the camera arm; identifying at least one of the feature sites of the at least one operating arm of the at least one operational image as at least one first feature site; obtaining the at least one control command from the input device, and obtaining a kinematics model of the at least one operating arm according to the at least one control command; obtaining at least one second feature site matching the at least one first feature site in the kinematics model; obtaining actual motion information of the at least one first feature site, and obtaining target motion information of the at least one second feature site; and comparing the actual motion information with the target motion information to determine whether there is a motion error in the surgical robot.

The program 505 can specifically be configured to make the processor 501 perform the following operations: obtaining at least one monitoring image of the camera arm and/or the at least one operating arm captured by the second image end instrument; identifying feature sites of the camera arm and/or the at least one operating arm of the at least one monitoring image as at least one first feature site; obtaining the at least one control command from the input device, and obtaining a kinematics model of the at least one operating arm according to the at least one control command; obtaining at least one second feature site matching the at least one first feature site in the kinematics model; and displaying the at least one second feature site on the display, comparing the at least one second feature site with the at least one first feature site to determine whether there is a motion error in the camera arm and/or the at least one operating arm.

Various technical features of the above-described embodiments may be combined in any combination, so that the description is concise, and all possible combinations of the various technical features in the above-described embodiments are described. However, as long as the combination of these technical features does not conflict, it is to be understood that the scope of the present specification is not to be taken in a limiting sense.

The above-described embodiments have only expressed several embodiments of the present application, which are described in more detail and detail, but are not therefore to be construed as limiting the scope of the present application. It should be noted that variations and modifications may be made to one of ordinary skill in the art without departing from the spirit of the present application, all of which fall within the scope of the present application. Therefore, the scope of the appended claims should be accorded the broadest interpretation so as to encompass all such modifications and similar arrangements. Therefore, the scope of protection of the patent application should be based on the appended claims.

## Claims

1. A surgical robot, **characterized in that**, comprises:
a camera arm;
at least one operating arm having more than one feature site;
an input device configured for receiving at least one control command for controlling the camera arm and/or the at least one operating arm to move; and
a control device operatively coupled to the camera arm and the at least one operating arm, the control device being configured for:
obtaining at least one operational image of a surgical field captured by the camera arm;
identifying at least one of the feature sites of the at least one operating arm as at least one first feature site;
obtaining the at least one control command from the input device, and obtaining a kinematics model of the at least one operating arm according to the at least one control command;
obtaining at least one second feature site matching the at least one first feature site in the kinematics model;
obtaining actual motion information of the at least one first feature site, and obtaining target motion information of the at least one second feature site, and comparing the actual motion information with the target motion information to determine whether there is a motion error in the surgical robot; or .

2. The surgical robot of claim 1, **characterized in that**, in the identifying the feature site of the at least one operating arm from the at least one operational image, the control device is configured for:
detecting whether at least one sub-image is comprised in the at least one operational image, wherein each of the at least one sub-image is at least part of the at least one operating arm (31);
extracting the at least one sub-image from the at least one operational image in response to the at least one sub-image being detected; and
identifying the feature site of the at least one operating arm based on the at least one sub-image.

3. The surgical robot of claim 2, **characterized in that**, in the detecting whether at least one sub-image is comprised in the at least one operational image, the control device is configured for:
inputting the at least one operational image into a neural network, and determining whether the at least one sub-image of the at least one operating arm is comprised in the at least one operational image through the neural network.

4. The surgical robot of claim 2, **characterized in that**, in the identifying the feature site of the at least one operating arm based on the at least one sub-image, the control device is configured for:
inputting the at least one sub-image into a neural network, and detecting the feature site of the at least one operating arm based on the at least one sub-image through the neural network.

5. The surgical robot of claim 1, **characterized in that**,
in the identifying the feature site of the at least one operating arm from the at least one operational image, the control device is configured for:
inputting at least one operational image into a neural network, and detecting the feature sites of at least one operating arm based on at least one operational image through the neural network.

6. The surgical robot of claim 4 or 5, **characterized in that**, in the obtaining the actual motion information of the at least one first feature site, the control device is configured for:
predicting the actual motion information of the at least one first feature site through the neural network.

7. The surgical robot of claim 6, **characterized in that**, the neural network is a convolutional neural network.

8. The surgical robot of claim 6, **characterized in that**, before the predicting the actual motion information of the at least one first feature site through the neural network, the control device is configured for:
training the convolutional neural network according to a difference between a prediction result and a real result, wherein the prediction result corresponds to the feature image extracted from a preset training-set through the convolutional neural network, and the real result corresponds to the preset training-set.

9. The surgical robot of claim 6, **characterized in that**, the actual motion information comprises actual position information and/or actual pose information.

10. The surgical robot of claim 6, **characterized in that**, the actual motion information comprises actual velocity information and/or actual angular velocity information.

11. The surgical robot of claim 1, **characterized in that**, an image end instrument with binocular vision is coupled to a distal end of the camera arm; in the obtaining the actual motion information of the at least one first feature site, the control device is configured for:
obtaining the actual motion information of the at least one first feature site under a viewpoint of image end instrument through a parallax principle, wherein the actual motion information is actual position information and/or orientation information.

12. The surgical robot according to claim 9 or 11, **characterized in that**, in the obtaining the target motion information of the at least one second feature site, the control device is configured for:
calculating the target motion information of the at least one second feature site according to the kinematics model, wherein the target motion information is target position information and/or target orientation information.

13. The surgical robot of claim 1, **characterized in that**, in the obtaining the kinematics model of the at least one operating arm according to the at least one control command, the control device is configured for:
obtaining target pose information of a distal end of the at least one operating arm by analyzing the at least one control command;
obtaining at least one joint variable of each joint assembly of the at least one operating arm by analyzing the target pose information; and
calculating the kinematics model of the at least one operating arm according to the at least one joint variable through a forward kinematics equation.

14. The surgical robot of claim 1, **characterized in that**, in the comparing the actual motion information with the target motion information to determine whether there is the motion error in the surgical robot, the control device is configured for:
calculating a deviation between the actual motion information and the target motion information;
judging whether the deviation exceeds a preset range; and
determining that there is a motion error in the surgical robot in response to the deviation exceeding the pre-set range; determining that there is no motion error in the surgical robot in response to the deviation not exceeding the pre-set range.

15. The surgical robot of claim 14, **characterized in that**, after the determining that there is a motion error in the surgical robot, the control device is configured for:
generating at least one motion error warning message.

16. The surgical robot of claim 15, **characterized in that**, the surgical robot comprises a display coupled to the control device, after the generating the at least one motion error warning message, the control device is configured for:
displaying the at least one motion error warning message on the display.

17. The surgical robot of claim 16, **characterized in that**, the displaying the at least one motion error warning message on the display, the control device is configured for:
generating at least one warning identification corresponding to the at least one motion error warning message in the at least one sub-image and displaying the at least one warning identification on the display.

18. The surgical robot of claim 17, **characterized in that**, the at least one warning identification comprises at least one contour line, and in the generating the at least one warning identification corresponding to the at least one motion error warning message in the at least one sub-image, the control device is configured for:
generating the at least one contour line at a contour of the at least one sub-image.

19. The surgical robot of claim 15, **characterized in that**, the surgical robot comprises an audio device coupled to the control device, after the generating the at least one motion error warning message, the control device is configured for:
generating auditory feedback corresponding to the at least one motion error warning message by the audio device.

20. The surgical robot of claim 15, **characterized in that**, the surgical robot comprises a haptic device coupled to the control device, after the generating the at least one motion error warning message, the control device is configured for:
generating haptic feedback corresponding to the at least one motion error warning message by the haptic device.

21. The surgical robot of claim 20, **characterized in that**, the haptic device is a vibration device, and the haptic feedback is reflected by a vibration; or the haptic device is a resistance device, and the haptic feedback is reflected by a resistance force.

22. The surgical robot of claim 21, **characterized in that**, in the generating haptic feedback corresponding to the at least one motion error warning message by the haptic device, the control device is configured for:
generating the haptic feedback corresponding to the at least one motion error warning message, wherein the haptic feedback is positively correlated with a degree of the deviation exceeding the pre-determined range.

23. The surgical robot of claim 14, **characterized in that**, after the determining that there is a motion error in the surgical robot, the control device is configured for:
generating at least one control command for forbidding a movement of the at least one operating arm to prevent the at least one operating arm from being controlled to move; or
generating at least one control command configured to prevent an input from the input device.

24. The surgical robot of claim 14, **characterized in that**, the input device is a mechanical handle, and each joint of the mechanical handle is equipped with a motor which drives the joint to move, after the determining that there is a motion error in the surgical robot, the control device is configured for:
generating at least one control command of increasing a torsion and/or torque of each motor to form a resistance force which prevents a movement of the mechanical handle.

25. The surgical robot of claim 1, **characterized in that**, before the obtaining the at least one second feature site(s) matching the at least one first feature site(s) in the kinematics model, the control device is configured for:
obtaining a weight value of each of the at least one first feature site;
weighting the first feature site according to the weight value of the first feature site to obtain a weighted value;
judging whether the weighted value reaches a start-up threshold; and
obtaining the at least one second feature site matching the at least one first feature site in the kinematics model in response to the weighted value reaching the start-up threshold.

26. The surgical robot of claim 1, **characterized in that**, each feature site comprises at least one prominent feature point which facilitates the identifying of the at least one feature site.

27. The surgical robot of claim 26, **characterized in that**, the at least one feature point comprises at least one of plane pattern, stereo geometry, and color.

28. The surgical robot of claim 27, **characterized in that**, the at least one feature point comprises at least one of point, line, and plane.

29. The surgical robot of claim 27, **characterized in that**, the at least one feature point comprises a graphic code with a pattern, and there is a correlation between the pattern and the feature sites, so that the at least one joint variable of the associated feature sites is obtained by calculating a zoomed state and/or a rotation state of the pattern under the viewpoint of the camera arm.

30. The surgical robot of claim 27, **characterized in that**, the at least one feature point comprises at least one of point, line, and plane.

31. The surgical robot of claim 26, wherein different feature sites are **characterized by** at least one different feature point.

32. The surgical robot of claim 1, wherein the feature sites comprise at least one of joint, linkage, and end instrument.

33. A surgical robot, **characterized in that**, comprises:
a camera arm;
at least one operating arm having more than one feature site;
an input device for inputting at least one control command for controlling the camera arm and/or the at least one operating arm to move; and
a control device operatively coupled to the camera arm and the at least one operating arm, the control device being configured for:
obtaining at least one operational image of a surgical field captured by the camera arm;
identifying feature sites of the at least one operating arm of the at least one operational image as at least one first feature site;
obtaining the at least one control command from the input device, and obtaining a kinematics model of the at least one operating arm according to the at least one control command;
obtaining at least one second feature site matching the at least one first feature site in the kinematics model;
and
displaying the at least one second feature site on the display, comparing the at least one second feature site with the at least one first feature site to determine whether there is a motion error in the surgical robot.

34. The surgical robot of claim 33, **characterized in that**, after identifying the feature sites of the at least one operating arm from the at least one operational image, the control device is configured for:
generating at least one first feature site identification identifying the at least one first feature site on the display.

35. The surgical robot of claim 34, **characterized in that**, in the generating the at least one first feature site identification on the display, the control device is configured for:
generating the at least one first feature site identification identifying the at least one first feature site in at least one sub-image.

36. The surgical robot of claim 34, **characterized in that**, an image end instrument with binocular vision is coupled to a distal end of the camera arm, in the generating the at least one first feature site identification identifying the at least one first feature site on the display, the control device is configured for:
obtaining actual position information and/or orientation information of the at least one first feature site under a viewpoint of image end instrument through a parallax principle;
reconstructing a 3D image of the at least one first feature site under the viewpoint of image end instrument based on the actual position information and/or posture information of the at least one first feature site and structural feature description information corresponding to the at least one first feature site; and
generating the at least one first feature site identification identifying the at least one first feature site in the 3D image, and the at least one first feature site identification is at a position corresponding to the at least one first feature site.

37. The surgical robot of claim 34, **characterized in that**, in the generating the at least one first feature site identification on the display, the control device is configured for:
obtaining position information and/or posture information of the at least one first feature site through a neural network;
reconstructing 3D image of the at least one first feature site under the viewpoint of image end instrument based on the actual position information and/or posture information of the at least one first feature site and structural feature description information corresponding to the at least one first feature site; and
generating at least one first feature site identification identifying the at least one first feature site in the 3D image, and the at least one first feature site identification is at a position corresponding to the at least one first feature site.

38. The surgical robot of claim 34, **characterized in that**, in the displaying the at least one second feature site on the display, the control device is configured for:
generating a model of the at least one second feature site under the viewpoint of image end instrument based on the calculated kinematics model of the at least one operating arm and structural feature description information of the at least one second feature site; and
generating at least one second feature site identification identifying the at least one second feature site in the model, and the at least one second feature site identification is at a position corresponding to the at least one second feature site.

39. The surgical robot of claim 34, **characterized in that**, in the identifying feature sites of the at least one operating arm from the at least one operational image, the control device is configured for:
inputting the at least one operational image into a neural network, and detecting feature sites of the at least one operating arm based on at least one operational image through the neural network.

40. The surgical robot of claim 39, **characterized in that**, after the detecting feature sites of the at least one operating arm based on the at least one operational image through the neural network, the control device is configured for:
predicting actual pose information of the feature sites through the neural network;
calculating a spacial model of an effective part of the feature sites based on the actual pose information of feature sites and a predetermined range;
generating a motion boundary to identify the feature sites, wherein the motion boundary is transformed from the spatial model and the motion boundary is at a position corresponding to the feature sites in the at least one sub-image under the viewpoint of the image end instrument.

41. The surgical robot of claim 40, **characterized in that**, the motion boundary of the feature sites comprises a plurality of substantially independent contours in response to the feature sites in the at least one operating arm being discontinuous parts; and the motion boundary of the feature sites comprises a whole contour in response to the feature sites in the at least one operating arm being continuous parts.

42. The surgical robot of claim 41, **characterized in that**, after the generating the motion boundary to identify the feature sites, the control device is configured for:
obtaining a first area of the motion boundary of the at least one first feature site under the viewpoint of image end instrument;
obtaining a second area of the at least one second feature site under the viewpoint of image end instrument, and the at least one second feature site is within or beyond the motion boundary of the at least one first feature site;
calculating a ratio of the second area with respect to the first area, and displaying the ratio on the display for determining whether there is a motion error in the surgical robot.

43. The surgical robot of claim 34, **characterized in that**, before the obtaining the at least one second feature site matching the at least one first feature site in the kinematics model, the control device is configured for:
obtaining a weight value of each of the first characteristics site;
weighting each of the first feature site according to the weight value of the first feature site to obtain a weighted value;
judging whether the weighted value reaches a start-up threshold; and
obtaining the at least one second feature site matching the at least one first feature site in the kinematics model in response to the weighted value reaching the start-up threshold.

44. A surgical robot, **characterized in that**, comprises:
a camera arm having a first image end instrument;
at least one operating arm having more than one feature site;
a second image end instrument;
an input device configured for receiving at least one control command for controlling the camera arm and/or the at least one operating arm to move; and
a control device operatively coupled to the camera arm, the at least one operating arm, the second image end instrument and the input device, the control device being configured for:
obtaining at least one monitoring image of the camera arm and/or the at least one operating arm captured by the second image end instrument;
identifying at least one of the feature sites of the camera arm and/or the at least one operating arm of the at least one monitoring image as at least one first feature site;
obtaining the at least one control command from the input device, and obtaining a kinematics model of the at least one operating arm according to the at least one control command;
obtaining at least one second feature site matching the at least one first feature site in the kinematics model;
obtaining actual motion information of the at least one first feature site, and obtaining target motion information of the at least one second feature site, and comparing the actual motion information with the target motion information to determine whether there is a motion error in the camera arm and/or the at least one operating arm.

45. A surgical robot, **characterized in that**, comprises:
a camera arm having a first image end instrument;
at least one operating arm having more than one feature site;
a second image end instrument having with respect to the camera arm and the at least one operating arm;
an input device for inputting at least one control command for controlling the camera arm and/or the at least one operating arm to move; and
a control device operatively coupled to the camera arm, the at least one operating arm, the second image end instrument and the input device, the control device being configured for:
obtaining at least one monitoring image of the camera arm and/or the at least one operating arm captured by the second image end instrument;
identifying feature sites of the camera arm and/or the at least one operating arm of the at least one monitoring image as at least one first feature site;
obtaining the at least one control command from the input device, and obtaining a kinematics model of the at least one operating arm according to the at least one control command;
obtaining at least one second feature site matching the at least one first feature site in the kinematics model;
and
displaying the at least one second feature site on the display, comparing the at least one second feature site with the at least one first feature site to determine whether there is a motion error in the camera arm and/or the at least one operating arm.

46. A motion error detection method for a surgical robot, wherein the surgical robot comprises a camera arm, at least one operating arm having more than one feature site and an input device configured for receiving at least one control command for controlling the camera arm and/or the at least one operating arm to move, comprising:
obtaining at least one operational image of a surgical field captured by the camera arm;
identifying at least one of the feature sites of the at least one operating arm of the at least one operational image as at least one first feature site;
obtaining the at least one control command from the input device, and obtaining a kinematics model of the at least one operating arm according to the at least one control command;
obtaining at least one second feature site matching the at least one first feature site in the kinematics model;
obtaining actual motion information of the at least one first feature site, and obtaining target motion information of the at least one second feature site; and
comparing the actual motion information with the target motion information to determine whether there is a motion error in the surgical robot.

47. A motion error detection method for a surgical robot, **characterized in that**, the surgical robot comprises a display, a camera arm, at least one operating arm having more than one feature site and an input device configured for receiving at least one control command for controlling the camera arm and/or the at least one operating arm to move, comprising:
obtaining at least one operational image of a surgical field captured by the camera arm;
identifying at least one of the feature sites of the at least one operating arm of the at least one operational image as at least one first feature site;
obtaining the at least one control command from the input device, and obtaining a kinematics model of the at least one operating arm according to the at least one control command;
obtaining at least one second feature site matching the at least one first feature site in the kinematics model;
displaying the at least one second feature site on the display, comparing the at least one second feature site with the at least one first feature site to determine whether there is a motion error in the surgical robot.

48. A motion error detection method for a surgical robot, **characterized in that**, the surgical robot comprises a camera arm having a first image end instrument, at least one operating arm having more than one feature site, a second image end instrument having with respect to the camera arm and the at least one operating arm, and an input device configured for receiving at least one control command for controlling the camera arm and/or the at least one operating arm to move, comprising:
obtaining at least one monitoring image of the camera arm and/or the at least one operating arm captured by the second image end instrument;
identifying at least one of the feature sites of the camera arm and/or the at least one operating arm of the at least one monitoring image as at least one first feature site;
obtaining the at least one control command from the input device, and obtaining a kinematics model of the at least one operating arm according to the at least one control command;
obtaining at least one second feature site matching the at least one first feature site in the kinematics model; obtaining actual motion information of the at least one first feature site, and obtaining target motion information of the at least one second feature site; and
comparing the actual motion information with the target motion information to determine whether there is a motion error in the camera arm and/or the at least one operating arm.

49. A motion error detection method for a surgical robot, **characterized in that**, the surgical robot comprises a camera arm having a first image end instrument, at least one operating arm having more than one feature site, a second image end instrument having with respect to the camera arm and the at least one operating arm, and an input device configured for receiving at least one control command for controlling the camera arm and/or the at least one operating arm to move, comprising:
obtaining at least one monitoring image of the camera arm and/or the at least one operating arm captured by the second image end instrument;
identifying feature sites of the camera arm and/or the at least one operating arm of the at least one monitoring image as at least one first feature site;
obtaining the at least one control command from the input device, and obtaining a kinematics model of the at least one operating arm according to the at least one control command;
obtaining at least one second feature site matching the at least one first feature site in the kinematics model;
and
displaying the at least one second feature site on the display, comparing the at least one second feature site with the at least one first feature site to determine whether there is a motion error in the camera arm and/or the at least one operating arm.

50. A computer readable storage medium, **characterized in that**, comprises:
a storage medium, storing a computer program configured to be loaded and executed by a processor to implement the detection method, described in any one of claims 46-49.

51. A motion error risk detection device for a surgical robot, comprising:
a memory for storing a computer program; and
a processor for loading and executing the computer program configured to be loaded and executed to implement the detection method described in any of claims 46-49.
